# EUROPÄISCHE PATENTSCHRIFT

(11) **EP 1 366 029 B1**
(45) Veröffentlichungstag und Bekanntmachung des Hinweises auf die Patenterteilung: **28.09.2005**
(21) Anmeldenummer: 02702317.5
(22) Anmeldetag: 28.01.2002
(51) Int. Cl.: C07D 263/20, A61K 31/422, A61P 7/02, C07D 413/10, C07D 413/14

(54) **SUBSTITUIERTE 2-OXO-3-PHENYL-5-CARBONYLAMINOMETHYL-1,3-OXAZOLINE UND IHRE VERWENDUNG ALS ANTIKOAGULANTIEN UND ANTITHROMBOTIKA**
SUBSTITUTED 2-OXO-3-PHENYL-5-CARBONYLAMINOMETHYL-1,3-OXAZOLINS AND THE UTILIZATION THEREOF AS ANTICOAGULANT AND ANTITHROMBOTIC AGENTS
2-OXO-3-PHENYL-5-CARBONYLAMINOMETHYL-1,3-OXAZOLINES SUBSTITUEES ET LEUR UTILISATION COMME ANTICOAGULANTS ET ANTITHROMBOTIQUES

(30) Priorität: 09.02.2001 DE 10105989
(43) Veröffentlichungstag der Anmeldung: 03.12.2003
(73) Patentinhaber: Bayer HealthCare AG, 51368 Leverkusen (DE)
(72) Erfinder: STRAUB, Alexander, 42117 Wuppertal (DE); LAMPE, Thomas, 40223 Düsseldorf (DE); PERNERSTORFER, Josef, 42103 Wuppertal (DE); PERZBORN, Elisabeth, 42327 Wuppertal (DE); POHLMANN, Jens, 42285 Wuppertal (DE); RÖHRIG, Susanne, 45276 Essen (DE); SCHLEMMER, Karl-Heinz, 42113 Wuppertal (DE)
(86) Internationale Anmeldenummer: PCT/EP2002/000857
(87) Internationale Veröffentlichungsnummer: WO 2002/064575

(56) Entgegenhaltungen:
- EP-A- 0 697 408
- WO-A-01/47919
- WO-A-99/31092

## Beschreibung

Die vorliegende Erfindung betrifft das Gebiet der Blutgerinnung. Insbesondere betrifft die vorliegende Erfindung neue Oxazolidinon-Derivate, Verfahren zu ihrer Herstellung sowie ihre Verwendung als Wirkstoffe in Arzneimitteln.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin, einem faserig-gallertigem Gerinnungsstoff. Darüber hinaus ist Thrombin ein potenter Auslöser der Thrombozytenaggregation, die ebenfalls einen erheblichen Beitrag bei der Hämostase leistet

Die Aufrechterhaltung der normalen Hämostase - zwischen Blutung und Thrombose - unterliegt einem komplexen Regulationsmechanismus. Die unkontrollierte Aktivierung des Gerinnungssystems oder eine defekte Hemmung der Aktivierungsprozesse kann die Bildung von lokalen Thromben oder Embolien in Gefäßen (Arterien, Venen, Lymphgefäßen) oder Herzhöhlen bewirken. Dies kann zu schwerwiegenden Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusslarankheiten, Lungenembolien oder tiefen venösen Thrombosen führen; diese Erkrankungen werden im folgenden zusammenfassend auch als thromboembolische Erkrankungen bezeichnet. Darüber hinaus kann eine Hyperkoagulabilität - systemisch - bei einer Verbrauchskoagulopathie zur disseminierten intravasalen Gerinnung führen.

Diese thromboembolischen Erkrankungen sind die häufigste Ursache von Morbidität und Mortalität in den meisten industrialisierten Ländern (Pschyrembel, Klinisches Wörterbuch, 257. Auflage, 1994, Walter de Gruyter Verlag, Seite 199 ff., Stichwort "Blutgerinnung"; Römpp Lexikon Chemie, Version 1.5, 1998, Georg Thieme Verlag Stuttgart, Stichwort "Blutgerinnung"; Lubert Stryer, Biochemie, Spektrum der Wissenschaft Verlagsgesellschaft mbH Heidelberg, 1990, Seiten 259 ff.).

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effigente Bebandlungsmethode bzw. Prävention von thromboembolischen Erkrankungen erweist sich in der Praxis deshalb als sehr schwierig und unbefriedigend.

Für die Therapie und Prävention von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen (Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin").

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben (Pschyrembel, Klinisches Wörterbuch, 257. Auflage, 1994, Walter de Gruyter Verlag, Seite 292 ff., Stichwort "Cumarinderivate"; Ullmann's Encyclopedia of Industrial Chemistry, 5. Auflage, VCH Verlagsgesellschaft, Weinheim, 1985 - 1996, Stichwort "Vitamin K").

In jüngster Zeit ist ein neuer Therapieansatz für die Behandlung und Prävention von thromboembolischen Erkrankungen beschrieben worden. Ziel dieses neuen Therapieansatzes ist die Inhibierung von Faktor Xa (vgl. WO-A-99/37304; WO-A-99/06371; J. Hauptmann, J. Stürzebecher, Thrombosis Research **1999,** *93,* 203; F. Al-Obeidi, J. A. Ostrem, Factor Xa inhibitors by classical and combinatorial chemistry, DDT **1998,** *3,* 223; F. Al-Obeidi, J. A. Ostrem, Factor Xa inhibitors, Exp. Opin. Ther. Patents **1999,** *9*, 931; B. Kaiser, Thrombin and factor Xa inhibitors, Drugs of the Future **1998,** *23,* 423; A. Uzan, Antithrombotic agents, Emerging Drugs **1998,** *3,* 189; B.-Y. Zhu, R. M. Scarborough, Curr. Opin. Card. Pulm. Ren. Inv. Drugs **1999**, *1 (1),* 63). Dabei ist gezeigt worden, dass verschiedene, sowohl peptidische wie nichtpeptidische Verbindungen in Tiermodellen als Faktor Xa-Inhibitoren wirksam sind.

WO9931092, EP0697408 und WO0147919 offenbaren strukturell verwandte Verbindungen der gleichen Wirkungsrichtung.

Aufgabe der vorliegenden Erfindung ist nunmehr die Bereitstellung neuer Substanzen zur Bekämpfung von Erkrankungen, die eine große therapeutische Bandbreite aufweisen.

Sie sollen insbesondere zur effizienteren Prävention und/oder Behandlung von thromboembolischen Erkrankungen geeignet sein und hierbei die zuvor geschilderten Nachteile des Standes der Technik - zumindest teilweise - vermeiden, wobei unter dem Begriff "thromboembolische Erkrankungen" im Sinne der vorliegenden Erfindung insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen verstanden werden.

Weitere Aufgabe der vorliegenden Erfindung ist die Bereitstellung neuer Antikoagulantien, welche mit erhöhter Selektivität den Blutgerinnungsfaktor Xa inhibieren und hierbei die Probleme der aus dem Stand der Technik bekannten Therapiemethoden für thromboembolische Erkrankungen - zumindest teilweise - vermeiden sollen.

Gegenstand der vorliegenden Erfindung sind Verbindungen der Formel (I) 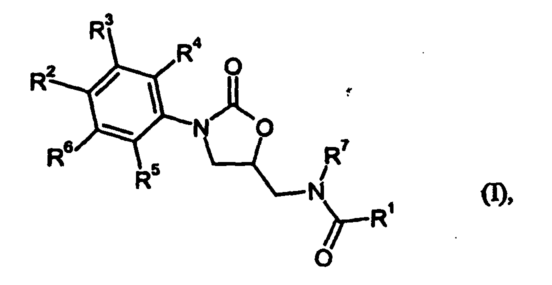 worin
- R¹: für (C₆-C₁₄)-Aryl, 5- bis 10-gliedriges Heteroaryl mit bis zu drei Heteroatomen aus der Reihe N, O und/oder S oder 5- bis 10-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Oxo, Carboxyl oder Cyano substituiert sein können,
- R²: für einen Rest -N(R¹⁰)C(O)R¹¹ oder 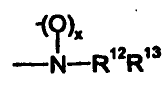 steht,
wobei
- R¹⁰ und R¹¹,: unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann,
(C₆-C₁₄)-Aryl, das seinerseits durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Carboxyl oder Cyano substituiert sein kann,
oder (C₃-C₇)-Cycloalkyl stehen,
oder
- R¹⁰ und R¹¹: gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, Trifluormethyl oder Cyano substituiert sein kann,
- x: für 0 oder 1 steht,
- R¹² und R¹³: gemeinsam mit dem Stickstoffatom, an das sie gebunden sind, einen 4- bis 6-gliedrigen Heterocyclus bilden, der noch ein weiteres Heteroatom aus der Reihe N, O und/oder S enthalten kann und der bis zu zweifach, unabhängig voneinander, durch Amino, Hydroxy, Halogen, Trifluormethyl, Cyano, Oxo, Mono- oder Di-(C₁- C₄)alkylamino, (C₁-C₄)-Alkoxy, Carboxamido, (C₁-C₄)-Alkylcarbonyl oder (C₃-C₅)-Cycloalkylcarbonyl substituiert sein kann,
- R³, R⁴, R⁵ und R⁶,: unabhängig voneinander, für Wasserstoff, Halogen, (C₁-C₆)-Alkyl, Amino, Mono- oder Di-(C₁-C₆)-alkylamino, Mono- oder Di-(C₁-C₆)-alkylaminocarbonyl, Hydroxy, (C₁-C₆)-Alkoxy, (C₁-C₆)-Alkanoyl, (C₁-C₆)-Alkanoylamino, Trifluormethyl, Carbamoyl, Nitro oder Cyano stehen,
und
- R⁷: für Wasserstoff oder (C₁-C₆)-Alkyl steht,
sowie deren Salze, Hydrate, Hydrate der Salze und Solvate,
ausgenommen jedoch Verbindungen der allgemeinen Formel (I), bei denen der Rest R¹ ein gegebenenfalls substituierter Thiophenrest ist.

Bislang sind Oxazolidinone im wesentlichen nur als Antibiotika, vereinzelt auch als MAO-Hemmer und Fibrinogen-Antagonisten beschrieben (Übersicht: Riedl, B., Endermann, R., Exp. Opin. Ther. Patents **1999,** *9* (5), 625), wobei für die antibakterielle Wirkung eine kleine 5-[Acyl-aminomethyl]-gruppe (bevorzugt 5-[Acetyl-aminomethyl]) essentiell zu sein scheint.

Substituierte Aryl- und Heteroarylphenyloxazolidinone, bei denen an das N-Atom des Oxazolidinonrings ein ein- oder mehrfach substituierter Phenylrest gebunden sein kann und die in der 5-Position des Oxazolidinonrings einen unsubstituierten N-Methyl-2-thiophencarboxamid-Rest aufweisen können, sowie ihre Verwendung als antibakteriell wirkende Substanzen sind bekannt aus den U.S.-Patentschriften US-A-5 929 248, US-A-5 801 246, US-A-5 756 732, US-A-5 654 435, US-A-5 654 428 und US-A-5 565 571.

Darüber hinaus sind benzamidinhaltige Oxazolidinone als synthetische Zwischenstufen bei der Synthese von Faktor Xa-Inhibitoren bzw. Fibrinogenantagonisten bekannt (WO-A-99/31092, EP-A-623615).

Die erfindungsgemäßen Verbindungen der allgemeinen Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der allgemeinen Formel (I) in tautomeren Formen vorliegen. Dies ist dem Fachmann bekannt, und derartige Verbindungen sind ebenfalls vom Umfang der Erfindung umfasst.

Salze der erfindungsgemäßen Verbindungen sind physiologisch unbedenkliche Salze der erfindungsgemäßen Verbindungen mit anorganischen oder organischen Säuren. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Salze können ebenso physiologisch unbedenkliche Salze mit üblichen Basen sein, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin oder Methylpiperidin.

Ausserdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Als "Hydrate" bzw. "Solvate" werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Halogen steht für Fluor, Chlor, Brom und Iod. Bevorzugt sind Chlor, Brom oder Fluor.

(C₁-C₆)-Alkyl steht für einen geradkettigen oder verzweigten Alkylrest mit 1 bis 6 Kohlenstoffatomen. Beispielsweise seien genannt: Methyl, Ethyl, n-Propyl, Isopropyl, n-Butyl, Isobutyl, tert.-Butyl, n-Pentyl und n-Hexyl. Aus dieser Definition leiten sich analog die entsprechenden Alkylgruppen mit weniger Kohlenstoffatomen wie z.B. (C₁-C₄)-Alkyl ab. Im allgemeinen gilt, dass (C₁-C₄)-Alkyl bevorzugt ist.

Aus dieser Definition leitet sich auch die Bedeutung des entsprechenden Bestandteils anderer komplexerer Substituenten ab wie z.B. bei Mono- oder Dialkylamino oder Mono- oder Di-alkylaminocarbonyl.

(C₃-C₇)-Cycloalkyl steht für einen cyclischen Alkylrest mit 3 bis 7 Kohlenstoffatomen. Beispielsweise seien genannt: Cyclopropyl, Cyclobutyl, Cyclopentyl, Cyclohexyl oder Cycloheptyl. Bevorzugt sind Cyclopropyl, Cyclopentyl und Cyclohexyl.

(C₁-C₆)-Alkoxy steht für einen geradkettigen oder verzweigten Alkoxyrest mit 1 bis 6 Kohlenstoffatomen. Beispielsweise seien genannt: Methoxy, Ethoxy, n-Propoxy, Isopropoxy, n-Butoxy, Isobutoxy, tert.-Butoxy, n-Pentoxy und n-Hexoxy. Aus dieser Definition leiten sich analog die entsprechenden Alkoxygruppen mit weniger Kohlenstoffatomen wie z.B. (C₁-C₄)-Alkoxy ab. Im allgemeinen gilt, dass (C₁-C₄)-Alkoxy bevorzugt ist.

(C₁-C₆)-Alkanoyl steht für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Beispielsweise seien genannt: Formyl, Acetyl, Propionyl, n-Butyryl, i-Butyryl, Pivaloyl, n-Hexanoyl. Aus dieser Definition leiten sich analog die entsprechenden Alkanoylgruppen mit weniger Kohlenstoffatomen wie z.B. (C₁-C₅)-Alkanoyl, (C₁-C₄)-Alkanoyl und (C₁-C₃)-Alkanoyl ab. Im allgemeinen gilt, dass (C₁-C₃)-Alkanoyl bevorzugt ist.

Aus dieser Definition leitet sich auch die Bedeutung des entsprechenden Bestandteils anderer komplexerer Substituenten ab wie z.B. Alkanoylamino.

(C₆-C₁₄)-Aryl steht für einen aromatischen Rest mit 6 bis 14 Kohlenstoffatomen. Beispielsweise seien genannt: Phenyl, Naphthyl, Phenanthrenyl und Anthracenyl. Aus dieser Definition leiten sich analog die entsprechenden Arylgruppen mit weniger Kohlenstoffatomen wie z.B. (C₆-C₁₀)-Aryl ab. Im allgemeinen gilt, dass (C₆-C₁₀)-Aryl bevorzugt ist.

5- bis 10-gliedriges Heteroaryl mit bis zu 3 Heteroatomen aus der Reihe N, O und/oder S steht für einen mono- oder bicyclischen gegebenenfalls benzokondensierten Heteroaromaten, der über ein Ringkohlenstoffatom oder Ringstickstoffatom des Heteroaromaten verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. Aus dieser Definition leiten sich analog die entsprechenden Heteroaromaten mit geringerer Ringgröße wie z.B. 5- bis 8-gliedriges Heteroaryl ab. Im allgemeinen gilt, dass 5- oder 6-gliedrige aromatische Heterocyclen wie z.B. Pyridyl, Pyridyl-N-oxid, Pyrimidyl, Pyridazinyl, Furyl und Thienyl bevorzugt sind.

5- bis 10-gliedriges Heterocyclyl mit bis zu 3 Heteroatomen aus der Reihe S, N, und/oder O steht für einen gesättigten oder teilweise ungesättigten, mono- oder bicyclischen, gegebenenfalls benzokondensierten Heterocyclus, der über ein Ringkohlenstoffatom oder ein Ringstickstoffatom verknüpft ist. Beispielsweise seien genannt: Tetrahydrofuryl, Pyrrolidinyl, Pyrrolinyl, Piperidinyl, 1,2-Dihydropyridinyl, 1,4-Dihydropyridinyl, Piperazinyl, Moipholinyl, Morpholinyl-N-oxid, Thiomorpholinyl, Azepinyl und 1,4-Diazepinyl. Bevorzugt sind Piperidinyl, Morpholinyl, Thiomorpholinyl und Pyrrolidinyl.

Aus dieser Definition leiten sich analog die entsprechenden Heterocyclen mit geringerer Ringgröße wie z.B. 4- bis 8-gliedrige Heterocyclen ab.

Bevorzugt sind Verbindungen der Formel (I),
worin
- R¹: für (C₆-C₁₄)-Aryl, 5- bis 10-gliedriges Heteroaryl mit einem Stickstoff- oder Sauerstoffatom als Heteroatom und gegebenenfalls bis zu zwei weiteren Heteroatomen aus der Reihe N, O und/oder S oder 5- bis 10-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Oxo, Carboxyl oder Cyano substituiert sein können,
- R²: für einen Rest -N(R¹⁰)C(O)R¹¹ oder 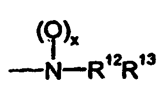 steht,
wobei
- R¹⁰ und R¹¹,: unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann,
(C₆-C₁₄)-Aryl, das seinerseits durch Halogen, (C₁-C₆)-Alkyl, Amino, Mono- oder Di-(C₁-C₆)-alkylamino, Hydroxy, (C₁-C₆)-Alkoxy, (C₁- C₆)-Alkoxycarbonyl, (C₁-C₆)-Alkanoyl, Trifluormethyl, Trifluormethoxy, Trifluormethylthio, Nitro, Carboxyl oder Cyano substituiert sein kann,
oder (C₃-C₇)-Cycloalkyl stehen,
oder
- R¹⁰: und R¹¹ gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, Trifluormethyl oder Cyano substituiert sein kann,
- x: für 0 oder 1 steht,
- R¹² und R¹³: gemeinsam mit dem Stickstoffatom, an das sie gebunden sind, einen 4- bis 6-gliedrigen Heterocyclus bilden, der noch ein weiteres Heteroatom aus der Reihe N, O und/oder S enthalten kann und der bis zu zweifach, unabhängig voneinander, durch Amino, Hydroxy, Halogen, Trifluormethyl, Cyano, Oxo, Mono- oder Di-(C₁- C₄)alkylamino, (C₁-C₄)-Alkoxy, Carboxamido, (C₁-C₄)-Alkylcarbonyl oder (C₃-C₅)-Cycloalkylcarbonyl substituiert sein kann,
- R³, R⁴, R⁵ und R⁶,: unabhängig voneinander, für Wasserstoff, Halogen, (C₁-C₆)-Alkyl, Amino, Mono- oder Di-(C₁-C₆)-alkylamino, Mono- oder Di-(C₁-C₆)-alkylaminocarbonyl, Hydroxy, (C₁-C₆)-Alkoxy, (C₁-C₆)-Alkanoyl, (C₁-C₆)-Alkanoylamino, Trifluormethyl, Carbamoyl, Nitro oder Cyano stehen,
und
- R⁷: für Wasserstoff oder (C₁-C₆)-Alkyl steht,
sowie deren Salze, Hydrate, Hydrate der Salze und Solvate.

Besonders bevorzugt sind Verbindungen der Formel (I),
worin
- R¹: für Phenyl, Naphtyl, 5- bis 8-gliedriges Heteroaryl mit einem Stickstoff- oder Sauerstoffatom als Heteroatom und gegebenenfalls bis zu zwei weiteren Heteroatomen aus der Reihe N, O und/oder S oder 5- bis 8-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, (C₁-C₆)-Alkoxy, Cyano, Trifluormethyl, Trifluormethoxy oder Trifluormethylthio substituiert sein können,
- R²: für einen Rest -N(R¹⁰)C(O)R¹¹ oder 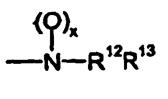 steht,
wobei
- R¹⁰ und R¹¹,: unabhängig voneinander, (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann, oder (C₃-C₇)-Cycloalkyl stehen,
oder
- R¹⁰ und R¹¹: gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, Trifluormethyl oder Cyano substituiert sein kann,
- x: für 0 oder 1 steht,
- R¹² und R¹³: gemeinsam mit dem Stickstoffatom, an das sie gebunden sind, einen 4- bis 6-gliedrigen Heterocyclus bilden, der noch ein weiteres Heteroatom aus der Reihe N, O und/oder S enthalten kann und der einfach durch Amino, Hydroxy, Halogen, Trifluormethyl, Cyano, Oxo, Mono- oder Di-(C₁-C₄)-alkylamino, (C₁-C₄)-Alkoxy, Carboxamido, (C₁-C₄)-Alkylcarbonyl oder (C₃-C₅)-Cycloalkylcarbonyl substituiert sein kann,
- R³ und R⁶,: unabhängig voneinander, für Wasserstoff, Halogen, (C₁-C₆)-Alkyl, Amino, Mono- oder Di-(C₁-C₆)-alkylamino, Hydroxy, (C₁-C₆)-Alkoxy, (C₁-C₆)-Alkanoylamino, Cyano, Trifluormethyl, oder Nitro stehen,
- R⁴ und R⁵: für Wasserstoff stehen,
und
- R⁷: für Wasserstoff oder (C₁-C₄)-Alkyl steht,
sowie deren Salze, Hydrate, Hydrate der Salze und Solvate.

Ganz besonders bevorzugt sind Verbindungen der Formel (I),
worin
- R¹: für Phenyl, Furyl, Dihydrothienyl, Thiazolyl, Pyrrolyl oder Pyridyl steht, wobei die Ringe ein- bis dreifach, unabhängig voneinander, durch Fluor, Chlor, Brom, (C₁-C₄)-Alkyl, Trifluormethyl, Trifluormethoxy oder Trifluormethylthio substituiert sein können,
- R²: für einen Rest -N(R¹⁰)C(O)R¹¹ oder 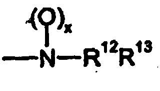 steht,
wobei
- R¹⁰ und R¹¹,: unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann, oder (C₃-C₇)-Cycloalkyl stehen,
oder
- R¹⁰ und R¹¹: gemeinsam mit der N-C(O)-Gruppe, an die sie gebunden sind, für Morpholinonyl, Pyrrolidinonyl, Thiomorpholinonyl oder Piperidinonyl stehen, wobei die Ringe ein- oder zweifach durch (C₁-C₄)-Alkyl substituiert sein können,
- x: für 0 oder 1 steht,
- R¹² und R¹³: gemeinsam mit dem Stickstoffatom, an das sie gebunden sind, einen 5- oder 6-gliedrigen gesättigten Heterocyclus bilden, der noch ein weiteres Sauerstoffatom im Ring enthalten kann und der einfach durch Amino oder Hydroxy substituiert sein kann,
- R³: für Wasserstoff, Fluor, Chlor, Brom, (C₁-C₄)-Alkyl, Amino, Mono- oder Di-(C₁-C₃)-alkylamino, Cyano oder Nitro stehen,
- R⁴, R⁵ und R⁶: für Wasserstoff stehen,
und
- R⁷: für Wasserstoff steht,
sowie deren Salze, Hydrate, Hydrate der Salze und Solvate.

Gegenstand der vorliegenden Erfindung ist auch ein Verfahren zur Herstellung der erfindungsgemäßen Verbindungen der allgemeinen Formel (I), wobei man

Verbindungen der Formel (II) 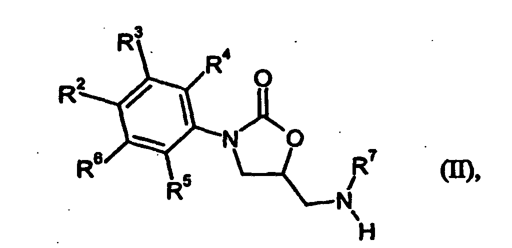 worin R², R³, R⁴, R⁵, R⁶ und R⁷ die oben angegebenen Bedeutungen haben,
mit Carbonsäuren der Formel (III) 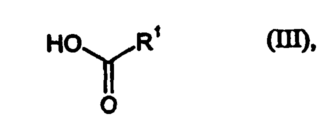 worin R¹ die oben angegebene Bedeutung hat,
oder aber mit den entsprechenden Carbonsäurehalogeniden, vorzugsweise Carbonsäurechloriden, oder aber mit den entsprechenden symmetrischen oder gemischten Carbonsäureanhydriden der zuvor definierten Carbonsäuren der Formel (III)
in inerten Lösungsmitteln, gegebenenfalls in Gegenwart von Hilfsstoffen und/oder Basen, zu Verbindungen der Formel (I) umsetzt.

Als Lösemittel für das zuvor beschriebenen Verfahren eignen sich hierbei organische Lösemittel, die unter den Reaktionsbedingungen inert sind. Hierzu gehören Halogenkohlenwasseistoffe wie Dichlormethan, Trichlormethan, Tetrachlormethan oder 1,2-Dichlorethan, Ether wie Diethylether, Dioxan oder Tetrahydrofuran, oder sonstige Lösemittel wie Ethylacetat, Dimethylformamid, Acetonitril, Pyridin, *N*-Methylpyrrolidon (NMP) oder Dimethylacetamid.

Ebenso ist es möglich, Lösemittelgemische der zuvor genannten Lösemittel einzusetzen.

Als Hilfsstoffe für die Amidbildung werden übliche Kondensationsmittel und/oder Aktivierungsreagenzien eingesetzt, wie Carbodiimide z.B. *N'*-(3-Dimethylaminopropyl)-*N*-ethylcarbodiimid · HCl (EDC), *N,N'*-Dicyclohexylcarbodiimid (DCC), gegebenenfalls in Gegenwart von 1-Hydroxy-1H-benzotriazol · H₂O (HOBt), Benzotriazol-1-yl-oxy-tris-pyrrolidino-phosphoniumhexafluorophosphat (PyBOP®), 2-(1*H*-Benzotriazol-1-yl)-1,1,3,3-tetramethyluroniumtetrafluoroborat (TBTU), 2-(1*H*-Benzotriazol-1-yl)-1,1,3,3-tetramethyluroniumhexafluorophosphat (HBTU), 2-(2-Oxo-1-(2*H*)-pyridyl)-1,1,3,3-tetramethyluroniumtetrafluoroborat (TPTU) oder O-(7-Azabenzotriazol-1-yl)-*N*,*N*,*N*',*N*'-tetramethyluroniumhexafluorophosphat (HATU) oder Carbonylverbindungen wie Carbonyldiimidazol.

Als Basen werden Trialkylamine z.B. Triethylamin, N-Methylmorpholin (NMM), N-Methylpiperidin, Diisopropylethylamin (Hünigbase) oder 4-*N,N*-Dimethylaminopyridin (DMAP) oder Pyridin eingesetzt.

Die Reaktionen erfolgen im allgemeinen in einem Temperaturbereich von 0°C bis zur Rückflusstemperatur, bevorzugt im Bereich von 0°C bis Raumtemperatur.

Die Umsetzungen können bei normalem, erhöhtem oder erniedrigtem Druck durchgeführt werden (z.B. im Bereich von 0,5 bis 5 bar). Im allgemeinen arbeitet man bei Normaldruck.

Die Verbindungen der allgemeinen Formeln (II) und (III) sind dem Fachmann an sich bekannt oder nach üblichen Methoden herstellbar. Für Oxazolidinone, insbesondere die benötigten 5-(Aminomethyl)-2-oxooxazolidine, vgl. WO-A-98/01446; WO-A-93/23384; WO-A-97/03072; J. A. Tucker et al., J. Med. Chem. **1998,** *41,* 3727; S. J. Brickner et al., J. Med. Chem. **1996,** *39,* 673; W. A. Gregory et al., J. Med. Chem. **1989,** *32,* 1673.

Die erfindungsgemäßen Verbindungen der allgemeinen Formel (I) zeigen ein nicht vorhersehbares, wertvolles pharmakologisches Wirkspektrum und sind daher insbesondere zur Prävention und/oder Behandlung von Erkrankungen geeignet.

Die erfindungsgemäßen Verbindungen der allgemeinen Formel (I) wirken insbesondere als Antikoagulantien und können daher bevorzugt eingesetzt werden in Arzneimitteln zur Prävention und/oder Behandlung von thromboembolischen Erkrankungen. Zu den "thromboembolischen Erkrankungen" im Sinne der vorliegenden Erfindung zählen insbesondere schwerwiegende Erkrankungen wie Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

Darüber hinaus sind die erfindungsgemäßen Verbindungen der allgemeinen Formel (I) gleichermaßen zur Behandlung der disseminierten intravasalen Gerinnung (DIC) geeignet.

Schließlich kommen die erfindungsgemäßen Verbindungen der allgemeinen Formel (I) ebenso für die Prävention und/oder Behandlung von Atherosklerose und Arthritis in Betracht, darüber hinaus ebenso für die Prävention und/oder Behandlung der Alzheimer'schen Erkrankung und von Krebs.

Weiterhin umfasst die vorliegende Erfindung auch ein Verfahren zur Verhinderung der Blutkoagulation in vitro, insbesondere bei Blutkonserven oder biologischen Proben, die Faktor Xa enthalten, das dadurch gekennzeichnet ist, dass Verbindungen der allgemeinen Formel (I) zugegeben werden.

Die erfindungsgemäßen Verbindungen der allgemeinen Formel (I) wirken insbesondere als selektive Inhibitoren des Blutgerinnungsfaktors Xa und hemmen nicht oder erst bei deutlich höheren Konzentrationen auch andere Serinproteasen wie Thrombin, Plasmin oder Trypsin.

Als "selektiv" werden im Rahmen der vorliegenden Erfindung solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Thrombin, Plasmin und Trypsin, um das 100-fache, vorzugsweise um das 500-fache, insbesondere um das 1.000-fache, kleiner sind, wobei bezüglich der Testmethoden für die Selektivität Bezug genommen wird auf die im folgenden beschriebenen Testmethoden der Beispiele A-1) a.1) und a.2).

Für die Applikation der erfindungsgemäßen Verbindungen kommen alle üblichen Applikationsformen in Betracht. Vorzugsweise erfolgt die Applikation oral, lingual, sublingual, bukkal, rektal oder parenteral (d.h. unter Umgehung des Intestinaltraktes, also intravenös, intraarteriell, intrakardial, intrakutan, subkutan, transdermal, intraperitoneal oder intramuskulär). Insbesondere geeignet sind die orale und intravenöse Applikation. Ganz besonders bevorzugt ist die orale Applikation, worin ein weiterer Vorteil gegenüber der aus dem Stand der Technik bekannten Therapie von thromboembolischen Erkrankungen liegt.

Die neuen Wirkstoffe der allgemeinen Formel (I) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,1 bis 95 Gew.-%, bevorzugt in 0,5 bis 90 Gew.-%, insbesondere von 1 bis 85 Gew.-%, der Gesamtmischung vorhanden sein, d.h. in Mengen, die ausreichend sind, um den angegebenen Dosierungsspielraum zu erreichen.

Trotzdem kann es gegebenenfalls erforderlich sein, von den zuvor genannten Mengen abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Im allgemeinen hat es sich als vorteilhaft erwiesen, bei intravenöser Applikation Mengen von etwa 0,001 bis 10 mg/kg, vorzugsweise etwa 0,01 bis 10 mg/kg, insbesondere etwa 0,1 bis 8 mg/kg Körpergewicht, zur Erzielung wirksamer Ergebnisse zu verabreichen.

Im allgemeinen hat es sich als vorteilhaft erwiesen, bei oraler Applikation Mengen von etwa 0,01 bis 50 mg/kg, vorzugsweise etwa 0,1 bis 10 mg/kg, insbesondere etwa 0,5 bis 8 mg/kg Körpergewicht, zur Erzielung wirksamer Ergebnisse zu verabreichen.

Trotzdem kann es gegebenenfalls erforderlich sein, von den zuvor genannten Mengen bei intravenöser bzw. oraler Applikation abzuweichen, und zwar in Abhängigkeit vom Körpergewicht bzw. von der Art des Applikationsweges, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese über den Tag zu verteilen, und zwar entweder in mehreren Einzelgaben oder als Dauerinfusion.

Die erfindungsgemäßen Verbindungen der allgemeinen Formel (I) zeichnen sich gegenüber herkömmlichen Präparaten zur Behandlung von thromboembolischen Erkrankungen insbesondere dadurch aus, dass durch die selektive Hemmung des Faktors Xa eine größere therapeutische Breite erreicht wird. Dies bedeutet für den Patienten ein geringeres Blutungsrisiko und für den behandelnden Arzt eine bessere Einstellbarkeit des Patienten. Außerdem erfolgt - durch den Mechanismus bedingt - ein schneller Wirkeintritt. Vor allem aber erlauben die erfindungsgemäßen Verbindungen eine orale Applikationsform, worin ein weiterer Vorteil der Therapie mit den erfindungsgemäßen Verbindungen liegt.

Die vorliegende Erfindung wird an den folgenden Beispielen veranschaulicht.

### A Bewertung der physiologischen Wirksamkeit

### 1. Allgemeine Testmethoden

Die besonders vorteilhaften biologischen Eigenschaften der erfindungsgemäßen Verbindungen können durch folgende Methoden festgestellt werden.

### a) Testbeschreibung (in vitro)

### a.1) Messung der Faktor Xa-Hemmung

Die enzymatische Aktivität von humanem Faktor Xa (FXa) wurde über die Umsetzung eines für den FXa-spezifischen chromogenen Substrats gemessen. Dabei spaltet der Faktor Xa aus dem chromogenen Substrat p-Nitroanilin ab. Die Bestimmungen wurden wie folgt in Mikrotiterplatten durchgeführt.

Die Prüfsubstanzen wurden in unterschiedlichen Konzentrationen in DMSO gelöst und für 10 Minuten mit humanem FXa (0,5 nmol/l gelöst in 50 mmol/l Tris-Puffer [C,C,C-Tris(hydroxymethyl)-aminomethan], 150 mmol/l NaCl, 0,1 % BSA (bovine serum albumine), pH = 8,3) bei 25°C inkubiert. Als Kontrolle dient reines DMSO. Anschließend wurde das chromogene Substrat (150 µmol/l Pefachrome® FXa von der Firma Pentapharm) hinzugefügt. Nach 20 Minuten Inkubationsdauer bei 25°C wurde die Extinktion bei 405 nm bestimmt. Die Extinktionen der Testansätze mit Prüfsubstanz wurden mit den Kontrollansätzen ohne Prüfsubstanz verglichen und daraus die IC₅₀-Werte berechnet.

| **Beispiel** | **IC**_{**50**} |
|---|---|
| 1 | 20 nM |
| 6 | 26 nM |

### a.2) Bestimmung der Selektivität

Zum Nachweis der selektiven FXa-Inhibition wurden die Prüfsubstanzen auf ihre Hemmung anderer humaner Serinproteasen wie Thrombin, Trypsin, Plasmin hin untersucht. Zur Bestimmung der enzymatischen Aktivität von Thrombin (75 mU/ml), Trypsin (500 mU/ml) und Plasmin (3,2 nmol/l) wurden diese Enzyme in Tris-Puffer (100 mmol/l, 20 mmol/l CaCl₂, pH = 8,0) gelöst und für 10 Minuten mit Prüfsubstanz oder Lösungsmittel inkubiert. Anschließend wurde durch Zugabe der entsprechenden spezifischen chromogenen Substrate (Chromozym Thrombin® von der Firma Boehringer Mannheim, Chromozym Trypsin® von der Firma Boehringer Mannheim, Chromozym Plasmin® von der Firma Boehringer Mannheim) die enzymatische Reaktion gestartet und die Extinktion nach 20 Minuten bei 405 nm bestimmt. Alle Bestimmungen wurden bei 37°C durchgeführt. Die Extinktionen der Testansätze mit Prüfsubstanz wurden mit den Kontrollproben ohne Prüfsubstanz verglichen und daraus die IC₅₀-Werte berechnet.

### a.3) Bestimmung der antikoagulatorischen Wirkung

Die antikoagulatorische Wirkung der Prüfsubstanzen wurde in vitro in Humanplasma bestimmt. Dazu wurde Humanblut unter Verwendung einer 0,11 molaren Natriumcitrat-Lösung als Vorlage in einem Mischungsverhältnis Natriumcitrat/Blut 1/9 abgenommen. Das Blut wurde unmittelbar nach der Abnahme gut gemischt und 10 Minuten bei ca. 2000 g zentrifugiert. Der Überstand wurde abpipettiert. Die Prothrombinzeit (PT, Synonyme: Thromboplastinzeit, Quick-Test) wurde in Gegenwart variierender Konzentrationen an Prüfsubstanz oder dem entsprechenden Lösungsmittel mit einem handelsüblichen Testkit (Neoplastin® von der Firma Boehringer Mannheim) bestimmt. Die Testverbindungen wurden 10 Minuten bei 37°C mit dem Plasma inkubiert. Anschließend wurde durch Zugabe von Thromboplastin die Gerinnung ausgelöst und der Zeitpunkt des Gerinnungseintritts bestimmt. Es wurde die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Ratte)

Nüchterne männliche Ratten (Stamm: HSD CPB:WU) mit einem Gewicht von 200 bis 250 g wurden mit einer Rompun/ Ketavet Lösung narkotisiert (12 mg/kg/50 mg/kg). Die Thrombusbildung wurde in einem arteriovenösen Shunt in Anlehnung an die von Christopher N. Beny et al., Br. J. Pharmacol. (1994), 113, 1209 bis 1214 beschriebene Methode ausgelöst. Dazu wurden die linke Vena jugularis und die rechte Arteria carotis freipräpariert. Ein extracorporaler Shunt wurde mittels eines 10 cm langen Polyethylenschlauchs (PE 60) zwischen den beiden Gefäßen gelegt. Dieser Polyethylenschlauch war in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthielt, eingebunden. Der extrakorporale Kreislauf wurde 15 Minuten lang aufrechterhalten. Dann wurde der Shunt entfernt und der Nylonfaden mit dem Thrombus sofort gewogen. Das Leergewicht des Nylonfadens war vor Versuchsbeginn ermittelt worden. Die Prüfsubstanzen wurden vor Anlegung des extrakorporalen Kreislaufs entweder intravenös über die Schwanzvene oder oral mittels Schlundsonde wachen Tieren verabreicht

### b.2) Arterielles Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Arteria carotis wurde freipräpariert (ca. 2 cm). Die Bildung eines arteriellen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die freipräparierte Arteria carotis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Anschließend wurde der Blutfluss durch einen um die Arteria carotis distal von dem verletzten Gefäßabschnitt gelegten Clip zusätzlich reduziert. Die proximale Klemme wurde entfernt, die Wunde verschlossen und nach 4 Stunden wieder geöffnet, um den verletzten Gefäßabschnitt zu entnehmen. Der Gefäßabschnitt wurde longitudinal geöffnet und der Thrombus von dem verletzten Gefäßabschnitt entfernt. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### b.3) Venöses Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Vena jugularis wurde freipräpariert (ca. 2 cm). Die Bildung eines venösen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die Vena jugularis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Der Blutfluss wurde wieder eröffnet und die Wunde verschlossen. Nach 4 Stunden wurde die Wunde wieder geöffnet, um die Thromben von den verletzten Gefäßabschnitten zu entfernen. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### B Herstellungbeispiele

### HPLC-Parameter:

[1] Säule: Kromasil C18 60*2, L-R Temperatur: 30°C, Fluß = 0.75 mlmin⁻¹, Eluent: A = 0.01 M H₃PO₄, B = CH₃CN, Gradient: ->0.5 min 90 %A -> 4.5 min 10 %A ->6.5 min 10 %A.
[2] Säule: Kromasil C18 60*2, L-R Temperatur: 30°C, Fluß = 0.75 mlmin⁻¹, Eluent: A = 0.005 M HClO₄, B = CH₃CN, Gradient: ->0.5 min 98 %A ->4.5 min 10 %A ->6.5 min 10 %A.
[3] Säule: Symmetry C18 2.1x150 mm, Säulenofen: 50°C, Fluß = 0.6 mlmin⁻¹, Eluent: A = 0.6 g 30%ige HCl/ 1 Wasser, B = CH₃CN, Gradient: 0.0 min 90 %A ->4.0 min 10%A -> 9 min 10 %A.
[4] MHZ-2P, Instrument Micromass Platform LCZ
   Säule Symmetry C18, 50 mm x 2.1 mm, 3.5 µm, Temperatur: 40°C, Fluss = 0.5 mlmin⁻¹, Eluent A = CH₃CN + 0.1 % Ameisensäure, Eluent B = Wasser + 0.1 % Ameisensäure, Gradient: 0.0 min 10 % A -> 4 min 90 % A -> 6 min 90 % A.

### Ausgangsverbindungen

### Beispiel I

### 4-(4-Morpholin-3-onyl)anilin

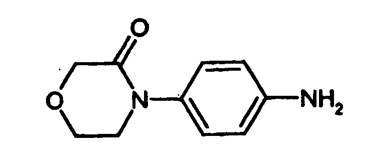

### I-a 4-(4-Morpholin-3-onyl)nitrobenzol

Die Darstellung von Morpholin-3-on wird in US-5,349,045 beschrieben.

Zu einer Lösung von Morpholin-3-on (202 g, 2 mol) in *N*-Methylpyrrolidon (21) wird über einen Zeitraum von 2 Stunden portionsweise Natriumhydrid (88 g, 2.2 mol, 60%ig in Paraffin) gegeben. Nach Beendigung der Wasserstoffentwicklung wird unter Kühlung 4-Fluornitrobenzol (282 g, 2 mol) innerhalb von einer Stunde zugetropft, und das Reaktionsgemisch wird über Nacht gerührt. Im Anschluß werden bei 12 mbar und 76°C 1,71 des Flüssigkeitsvolumens abdestilliert, der Rückstand wird auf Wasser (21) gegossen und dieses Gemisch zweimal mit Ethylacetat (je 1 1) extrahiert. Die vereinigten organischen Phasen werden mit Wasser gewaschen, über Natriumsulfat getrocknet, filtriert und im Vakuum eingeengt. Die Reinigung erfolgt durch Chromatographie (an Kieselgel, Hexan-Ethylacetat 1:1) und nachfolgende Kristallisation aus Ethylacetat. Ausbeute: 78 g (farbloser bis bräunlicher Feststoff), 17.6 % der Theorie.
¹H-NMR (300 MHz, CDCl₃): 3.86 (m, 2 H, C*H*₂CH₂), 4.08 (m, 2 H, CH₂C*H*₂), 4.49 (s, 2 H, C*H*₂CO), 7.61 (d, 2 H, ³*J*=8.95 Hz, C*H*CH), 8,28 (d, 2 H, ³*J*=8.95 Hz, CHC*H*);
MS (r.I.%) = 222 (74, M⁺), 193 (100), 164 (28), 150 (21), 136 (61), 117 (22), 106 (24), 90 (37), 76 (38), 63 (32), 50 (25).

### I-b 4-(4-Morpholin-3-onyl)anilin

In einem Autoklaven wird 4-(4-Morpholin-3-onyl)nitrobenzol (63 g, 0.275 mol) in Tetrahydrofuran (200 ml) gelöst, mit Pd/C (3.1 g, 5 %ig) versetzt und 8 Stunden bei 70°C und einem Wasserstoffdruck von 50 bar hydriert. Nach Filtration des Katalysators wird das Lösemittel im Vakuum abdestilliert und das Produkt durch Kristallisation aus Ethylacetat gereinigt. Ausbeute: 20 g (farbloser bis bläulicher Feststoff), 37.6 % der Theorie. Die Reinigung kann alternativ auch durch Chromatographie (an Kieselgel, Hexan-Ethylacetat-Gemisch) erfolgen.
¹H-NMR (300 MHz, CDCl₃: 3.67 (m, 2 H, C*H*CH₂, 3.999 (m, CH₂C*H*₂), 4.27 (s, 2 H, C*H*₂CO), 6.68 (d, 2 H, ³*J*=8,71 Hz, C*H*CH), 7.03 (d, 2 H, ³*J*=8,71 Hz, CHC*H*);
MS (r.I.%) = 192 (100, M⁺), 163 (48), 133 (26), 119 (76), 106 (49), 92 (38), 67 (27), 65 (45), 52 (22), 28 (22).

### Beispiel II

### 4-{4-[(5S)-5-(Aminomethyl)-2-oxo-1,3-oxazolidin-3-yl]phenyl}-3-morpholinon

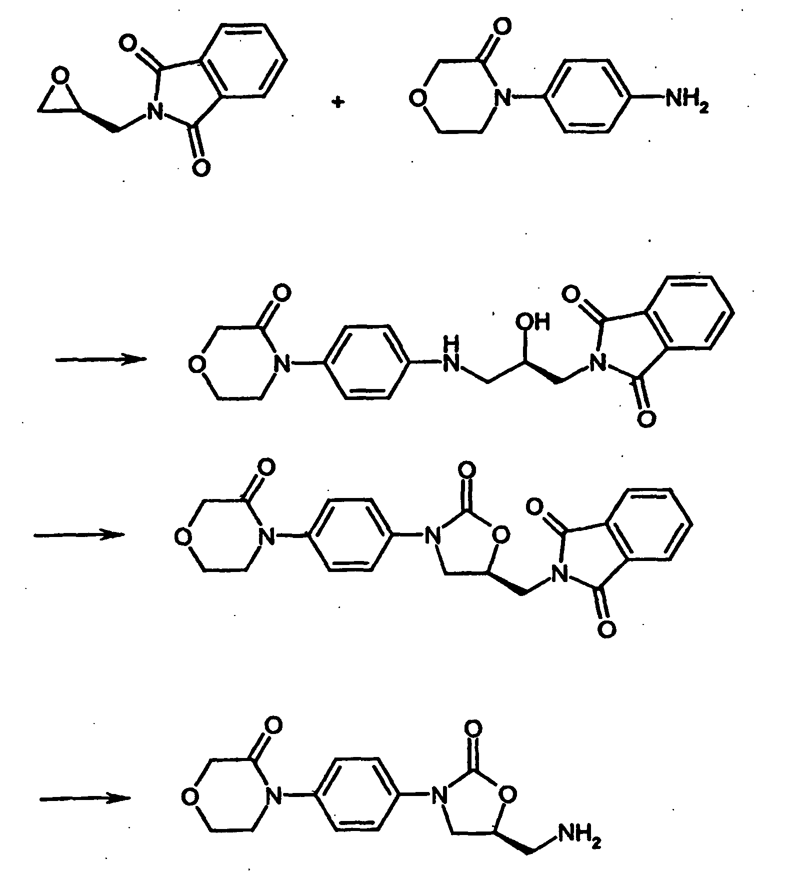

### II-a 2-((2R)-2-Hydroxy-3-{[4-(3-oxo-4-morpholinyl)phenyl]amino}propyl)-1H-isoindol-1,3(2H)-dion

Eine Suspension von 2-[(2*S*)-2-Oxiranylmethyl]-1*H*-isoindol-1,3(2*H*)-dion (A. Gutcait *et al. Tetrahedron Asym*. **1996,** *7,* 1641) (5.68 g, 27.9 mmol) und 4-(4-Aminophenyl)-3-morpholinon (5.37 g, 27.9 mmol) in Ethanol-Wasser (9:1, 140 ml) wird für 14 Stunden unter Rückfluss erhitzt (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Die vereinigten Mutterlaugen werden im Vakuum eingeengt und nach Zugabe einer zweiten Portion 2-[(2*S*)-2-Oxiranylmethyl]-1*H*-isoindol-1,3(2*H*)-dion (2.84 g, 14.0 mmol) in Ethanol-Wasser (9:1, 70 ml) suspendiert und für 13 Stunden unter Rückfluss erhitzt (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Gesamtausbeute : 10.14 g, 92 % der Theorie.
MS (ESI): m/z (%) = 418 ([M+Na]⁺, 84), 396 ([M+H]⁺, 93);
HPLC (Methode 2): rt = 3.34 min.

### II-b 2-({(5S)-2-Oxo-3-[4-(3-oxo-4-morpholinyl)phenyl]-1,3-oxazolidin-5-yl}methyl)-1H-isoindol-1,3(2H)-dion

Zu einer Suspension des Aminoalkohols (3.58 g, 9.05 mmol) in Tetrahydrofuran (90 ml) wird unter Argon bei Raumtemperatur *N,N'*-Carbonyldiimidazol (2.94 g, 18.1 mmol) und Dimethylaminopyridin (katalytische Menge) gegeben. Die Reaktionssuspension wird bei 60°C für 12 Stunden gerührt (der Niederschlag geht in Lösung, nach einiger Zeit erneute Bildung eines Niederschlages), mit einer zweiten Portion *N,N'*-Carbonyldiimidazol (2.94 g, 18.1 mmol) versetzt und weitere 12 Stunden bei 60°C gerührt. Der Niederschlag (gewünschtes Produkt) wird abfiltriert, mit Tetrahydrofuran gewaschen und getrocknet. Das Filtrat wird im Vakuum eingeengt und weiteres Produkt mittels Flash-Chromatographie (Dichlormethan-Methanol-Gemische) gereinigt. Gesamtausbeute: 3.32 g, 87 % der Theorie.
MS (ESI): m/z (%) = 422 ([M+H]⁺, 100);
HPLC (Methode 3): rt = 3.37 min.

### II-c 4-{4-[(5S)-5-(Aminomethyl)-2-oxo-1,3-oxazolidin-3-yl]phenyl}-3-morpholinon

Zu einer Suspension des Oxazolidinons (4.45 g, 10.6 mmol) in Ethanol (102 ml) wird bei Raumtemperatur tropfenweise Methylamin (40%ig in Wasser, 10.2 ml, 0.142 mol) gegeben. Die Reaktionsmischung wird für 1 Stunde unter Rückfluss erhitzt und im Vakuum eingeengt. Das Rohprodukt wird ohne weitere Reinigung in die nächste Reaktion eingesetzt.
MS (ESI): m/z (%) = 292 ([M+H]⁺, 100);
HPLC (Methode 2): rt = 2.66 min.

### Beispiel III

### (5S)-5-(Aminomethyl)-3-[4-(2-oxo-1-pyrrolidinyl)phenyl]-1,3-oxazolidin-2-on

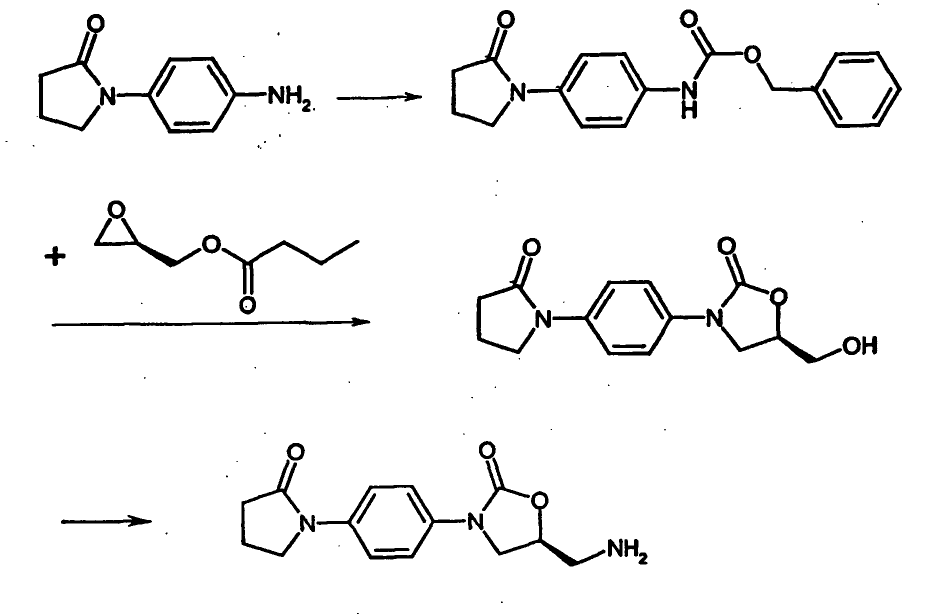

### III-a Benzyl 4-(2-oxo-1-pyrrolidinyl)phenylcarbamat

1-(4-Aminophenyl)pyrrolidin-2-on (4 g, 22.7 mmol) und *N,N*-Dimethylanilin (3.6 ml, 28.4 mmol) werden in Tetrahydrofuran (107 ml) bei -20°C langsam mit Chlorameisensäurebenzylester (4.27 g, 25.03 mmol) versetzt. Das Reaktionsgemisch wird 30 min bei -20°C gerührt und anschließend auf Raumtemperatur erwärmt. Nach Zugabe von Ethylacetat (0.5 l) wird die organische Phase mit gesättigter NaCl-Lösung (0.5 l) gewaschen. Die vereinigten organische Phase werden getrocknet (Magnesiumsulfat), filtriert und im Vakuum eingeengt. Der Rückstand wird mit Diethylether verrieben und abgesaugt. Ausbeute: 5.2 g, 74 % der Theorie, hellbeige Kristalle, Schmelzpunkt: 174°C.

### III-b (5R)-5-(Hydroxymethyl)-3-[4-(2-oxo-1-pyrrolidinyl)phenyl]-1,3-oxazolidin-2-on

Isoamylalkohol (1.47 g, 16.66 mmol) in Tetrahydrofuran (200 ml) wird unter Argon bei -10°C tropfenweise mit n-Butyllithium (7.27 ml, 2.5 M Lösung in Hexan) versetzt, wobei weiteres n-Butyllithium bis zum Umschlag des hinzugesetzten Indikators N-Benzylidenbenzylamin hinzugegeben wird. Das Reaktionsgemisch wird 10 Minuten bei -10°C gerührt, auf -78°C gekühlt und langsam mit einer Lösung von Benzyl-4-(2-oxo-1-pyrrolidinyl)phenylcarbamat (4.7 g, 15.14 mmol) versetzt. Anschließend wird nochmals bis zum Farbumschlag des Indikators nach rosa n-Butyllithium (2.5 M Lösung in Hexan) hinzugegeben. Das Reaktionsgemisch wird 10 Minuten bei -78°C gerührt, mit *R*-Glycidylbutyrat (2.62 g, 18.17 g) versetzt und für 30 Minuten bei -78°C gerührt. Das Reaktionsgemisch wird über Nacht auf Raumtemperatur erwärmt und mit Wasser (200 ml) versetzt. Der Tetrahydrofuran-Anteil wird im Vakuum entfernt. Der wäßrige Rückstand wird mit Ethylacetat extrahiert, die vereinigten organische Phasen mit (Magnesiumsulfat) getrocknet, filtriert und im Vakuum eingeengt. Der Rückstand wird mit Diethylether (500 ml) verrieben, und die ausgefallenen Kristalle abgesaugt. Ausbeute: 3.76 g, 90 % der Theorie.
Schmelzpunkt: 148°C,
R_{f} (SiO₂, Toluol-Ethylacetat 1:1) = 0.04, (Edukt = 0.3).

### III-c (5S)-5-(Aminomethyl)-3-[4-(2-oxo-1-pyrrolidinyl)phenyl]-1,3-oxazolidin-2-on

Zu einer Lösung von (5*R*)-5-(Hydroxymethyl)-3-[4-(2-oxo-1-pyrrolidinyl)phenyl]-1,3-oxazolidin-2-on (3.6 g, 13.0 mmol) und Triethylamin (2.9 g, 28.7 mmol) in Dichlormethan (160 ml) wird bei 0°C Methansulfonsäurechlorid (1.79 g, 15.64 mmol) gegeben. Das Reaktionsgemisch wird 1.5 Stunden bei 0°C sowie 3 Stunden bei Raumtemperatur gerührt, mit Wasser gewaschen, und die wäßrige Phase wird nochmals mit Dichlormethan extrahiert. Die vereinigten organischen Phasen werden getrocknet (Magnesiumsulfat), filtriert und im Vakuum eingeengt. Anschließend wird der Rückstand (1.67 g) in Acetonitril (70 ml) gelöst, mit Phthalimidkalium (2.62 g,14.16 mmol) versetzt und in einem geschlossenen Gefäß in einem Mikrowellenofen 45 min lang bei 180°C gerührt. Das Gemisch wird durch Filtration von unlöslichem Rückstand befreit, das Filtrat im Vakuum eingedampft, der Rückstand (1.9 g) in Methanol gelöst und mit Hydrazinhydrat (0.47 g, 9.37 mmol) versetzt. Das Reaktionsgemisch wird 2 Stunden refluxiert, abgekühlt, mit gesättigter Natriumbicarbonatlösung versetzt und sechsmal mit Methylenchlorid (insgesamt 2 1) extrahiert. Die vereinigten organischen Phasen werden getrocknet (Magnesiumsulfat), filtriert und im Vakuum eingeengt.
Das erhaltene Produkt wird ohne weitere Reinigung eingesetzt.

### Synthesebeispiele

### Beispiel 1

### 4-Chlor-N-({(5S)-2-oxo-3-[4-(3-oxo-4-morpholinyl)phenyl]-1,3-oxazolidin-5-yl}methyl)benzamid

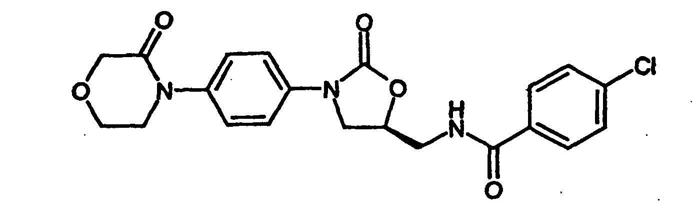

Zu einer Lösung des Amins II (80.0 mg, 0.27 mmol) in Pyridin (2 ml) wird unter Argon bei Raumtemperatur 4-Chlorbenzoylchlorid (72.1 mg, 0.41 mmol) getropft. Das Reaktionsgemisch wird 2 Stunden bei Raumtemperatur gerührt, mit Pyridin (4 ml) verdünnt, mit Aminomethylpolystyrolharz (2.5 eq.) versetzt und für 1.5 Stunden bei Raumtemperatur geschüttelt. Das Harz wird filtriert und mehrmals mit Dichlormethan-Methanol (5:1) gewaschen. Die vereinigten Filtrate werden im Vakuum eingeengt. Das gewünschte Produkt wird mittels Flash-Chromatographie (Dichlormethan-Methanol-Gemische) gereinigt. Ausbeute: 105.1 mg, 89% der Theorie.
¹H NMR (DMSO-d⁶, 200 MHz): 8.90 (t, 1H), 7.86 (d, 2H), 7.60-7.50 (m, 4H), 7.40 (d, 2H), 4.92-4.82 (m, 1H), 4.24-4.15 (m, 1H), 4.19 (s, 2H), 4.00-3.93 (m, 2H), 3.93-3.85 (dd, 1H), 3.75-3.68 (m, 2H), 3.68-3.60 (m, 2H);
MS (DCI, NH₃): m/z (%) = 447 ([M+NH₄]⁺, 100);
HPLC (Methode 2): rt = 3.76 min.

Folgende Verbindungen wurden analog dargestellt:

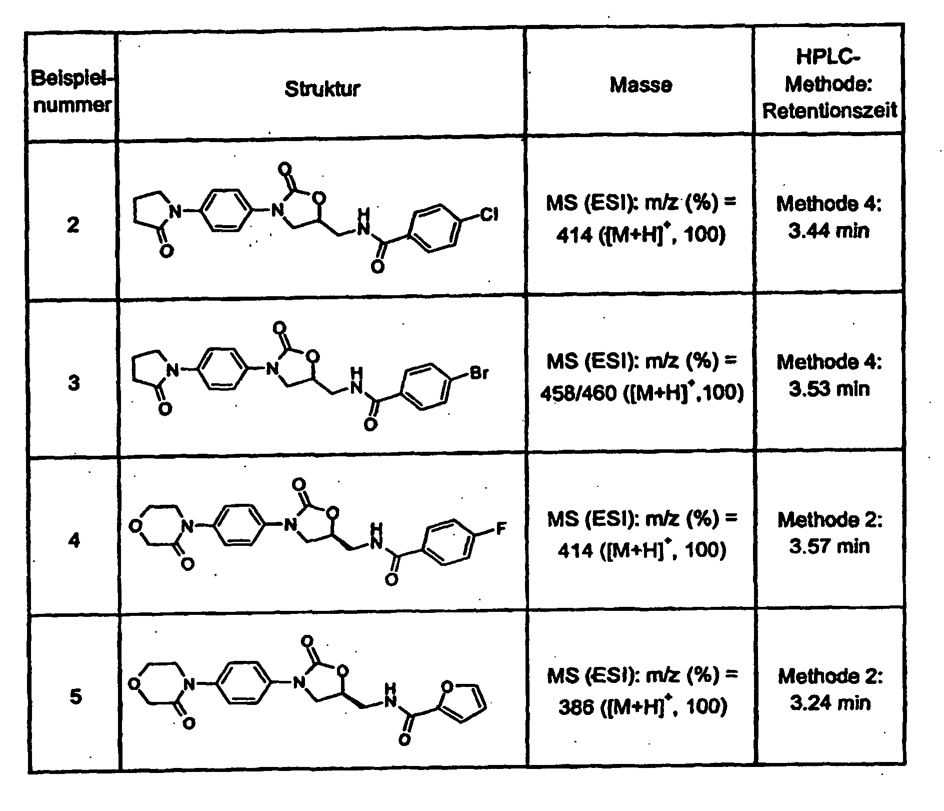

### Beispiel 6

### 5-Brom-N-({(5S)-2-oxo-3-[4-(3-oxo-4-morpholinyl)phenyl]-1,3-oxazolidin-5-yl}methyl)-2-furamid

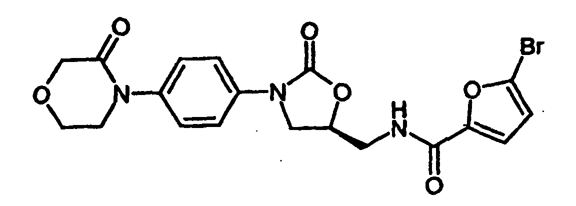

Eine Lösung des Amins II (100.0 mg, 0.34 mmol), 5-Brom-2-furancarbonsäure (78.7 mg, 0.41 mmol) und 1-Hydroxy-1*H*-benzotriazol (55.7 mg, 0.41 mmol) in Dimethylformamid (3.4 ml) wird unter Argon bei Raumtemperatur mit *N*-(3-Dimethylaminopropyl)-ethylcarbodiimid-hydrochlorid (79.0 mg, 0.41 mmol) und tropfenweise mit *N,N*-Diisopropylethylamin (120 µl, 0.69 mmol) versetzt. Das Reaktionsgemisch wurde 16 Stunden bei Raumtemperatur gerührt und im Vakuum eingeengt. Das gewünschte Produkt wird mittels Flash-Chromatographie (Dichlormethan-Methanol-Gemische) gereinigt. Ausbeute: 79.6 mg, 50 % der Theorie.
¹H NMR (DMSO-d⁶, 200 MHz): 8.79 (t, 1H), 7.56 (d, 2H), 7.41 (d, 2H), 7.19 (d, 1H), 6.77 (d, 1H), 4.88-4.77 (m, 1H), 4.19 (s, 2H), 4.18 (dd, 1H), 4.00-3.93 (m, 2H), 3.90-3.82 (dd, 1H), 3.75-3.67 (m, 2H), 3.62-3.53 (m, 2H);
MS (ESI): m/z (%) = 464 ([M+H]⁺, 100);
HPLC (Methode 1): rt = 3.31 min.

Folgende Verbindungen wurden analog dargestellt:

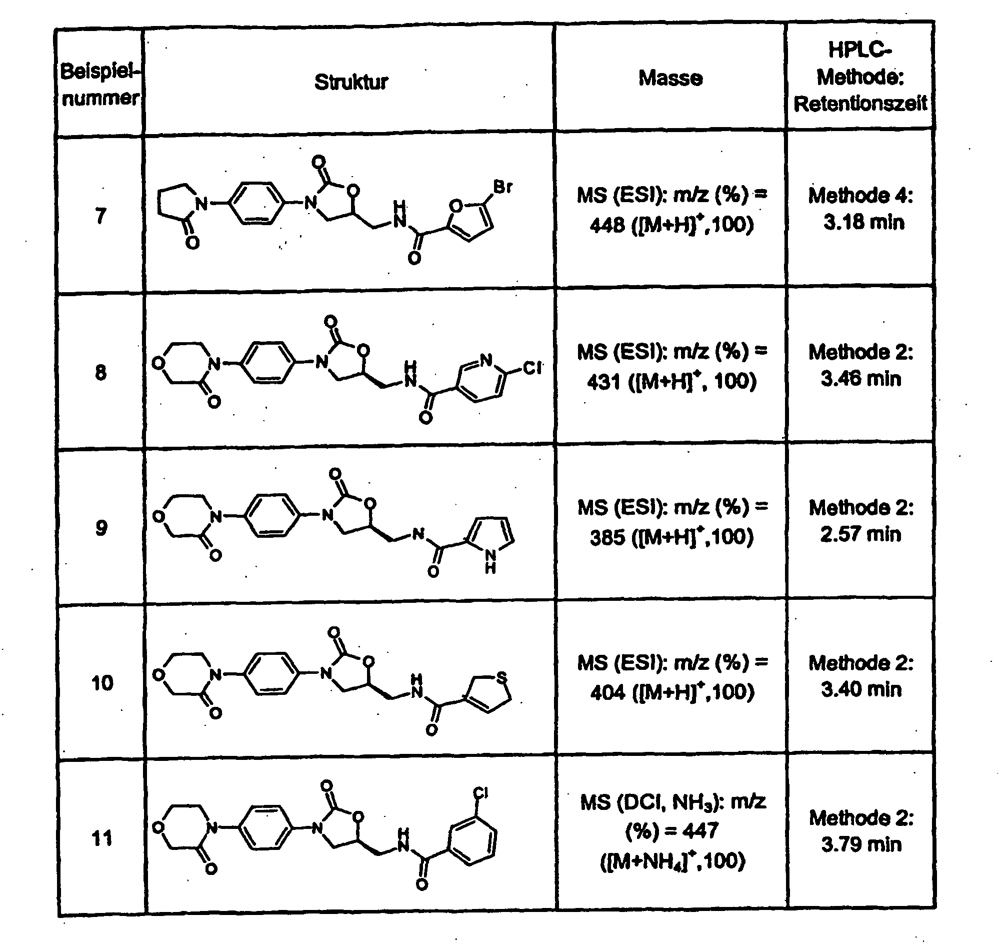

## Patentansprüche

1. Verbindungen der Formel (I) 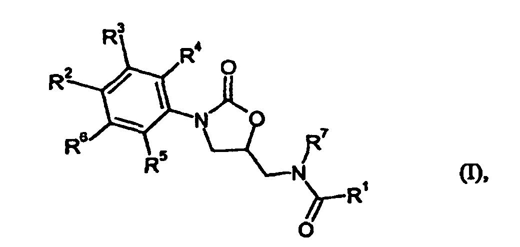 worin
R¹ für (C₆-C₁₄)-Aryl, 5- bis 10-gliedriges Heteroaryl mit bis zu drei Heteroatomen aus der Reihe N, O und/oder S oder 5- bis 10-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Oxo, Carboxyl oder Cyano substituiert sein können,
R² für einen Rest -N(R¹⁰)C(O)R¹¹ oder 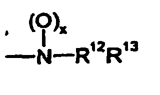 steht,
wobei
R¹⁰ und R¹¹, unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann,
(C₆-C₁₄)-Aryl, das seinerseits durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Carboxyl oder Cyano substituiert sein kann,
oder (C₃-C₇)-Cycloalkyl stehen,
oder
R¹⁰ und R¹¹ gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono-oder Di-(C₁-C₆)-alkylamino, Hydroxy, (C₁-C₆)-Alkoxy, Trifluormethyl oder Cyano substituiert sein kann,
x für 0 oder 1 steht,
R¹² und R¹³ gemeinsam mit dem Stickstoffatom, an das sie gebunden sind, einen 4- bis 6-gliedrigen Heterocyclus bilden, der noch ein weiteres Heteroatom aus der Reihe N, O und/oder S enthalten kann und der bis zu zweifach, unabhängig voneinander, durch Amino, Hydroxy, Halogen, Trifluormethyl, Cyano, Oxo, Mono- oder Di-(C₁-C₄)-alkylamino, (C₁-C₄)-Alkoxy, Carboxamido, (C₁-C₄)-Alkylcarbonyl oder (C₃-C₅)-Cycloalkylcarbonyl substituiert sein kann,
R³, R⁴, R⁵ und R⁶, unabhängig voneinander, für Wasserstoff, Halogen, (C₁-C₆)-Alkyl, Amino, Mono- oder Di-(C₁-C₆)-alkylamino, Mono-oder Di-(C₁-C₆)-alkylaminocarbonyl, Hydroxy, (C₁-C₆)-Alkoxy, (C₁-C₆)-Alkanoyl, (C₁-C₆)-Alkanoylamino, Trifluormethyl, Carbamoyl, Nitro oder Cyano stehen,
und
R⁷ für Wasserstoff oder (C₁-C₆)-Alkyl steht,
sowie deren Salze, Hydrate, Hydrate der Salze und Solvate,
ausgenommen jedoch Verbindungen der allgemeinen Formel (I), bei denen der Rest R¹ ein gegebenenfalls substituierter Thiophenrest ist.

2. Verbindungen der Formel (I) nach Anspruch 1,
worin
R¹ für (C₆-C₁₄)-Aryl, 5- bis 10-gliedriges Heteroaryl mit einem Stickstoff- oder Sauerstoffatom als Heteroatom und gegebenenfalls bis zu zwei weiteren Heteroatomen aus der Reihe N, O und/oder S oder 5-bis 10-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, Amino, Mono-oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Oxo, Carboxyl oder Cyano substituiert sein können,
R² für einen Rest -N(R¹⁰)C(O)R¹¹ oder 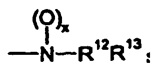 steht,
wobei
R¹⁰ und R¹¹, unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann,
(C₆-C₁₄)-Aryl, das seinerseits durch Halogen, (C₁-C₆)-Alkyl, Amino, Mono- oder Di-(C₁-C₆)-alkylamino, Hydroxy, (C₁-C₆)-Alkoxy, (C₁-C₆)-Alkoxycarbonyl, (C₁-C₆)-Alkanoyl, Trifluormethyl, Trifluormethoxy, Trifluormethylthio, Nitro, Carboxyl oder Cyano substituiert sein kann,
oder (C₃-C₇)-Cycloalkyl stehen,
oder
R¹⁰ undR ¹¹ gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono-oder Di-(C₁-C₆)-alkylamino, Hydroxy, (C₁-C₆)-Alkoxy, Trifluormethyl oder Cyano substituiert sein kann,
x für 0 oder 1 steht,
R¹² und R¹³ gemeinsam mit dem Stickstoftatom, an das sie gebunden sind, einen 4- bis 6-gliedrigen Heterocyclus bilden, der noch ein weiteres Heteroatom aus der Reihe N, O und/oder S enthalten kann und der bis zu zweifach, unabhängig voneinander, durch Amino, Hydroxy, Halogen, Trifluormethyl, Cyano, Oxo, Mono- oder Di-(C₁-C₄)-alkylamino, (C₁-C₄)- Alkoxy, Carboxamido, (C₁-C₄)-Alkylcarbonyl oder (C₃-C₅)- Cycloalkylcarbonyl substituiert sein kann,
R³,R⁴, R⁵ und R⁶, unabhängig voneinander, für Wasserstoff, Halogen, (C₁-C₆)-Alkyl, Amino, Mono- oder Di-(C₁-C₆)-alkylamino, Mono-oder Di-(C₁-C₆)-alkylaminocarbonyl, Hydroxy, (C₁-C₆)-Alkoxy, (C₁-C₆)-Alkanoyl, (C₁-C₆)-Alkanoylamino, Trifluormethyl, Carbamoyl, Nitro oder Cyano stehen,
und
R⁷ für Wasserstoff oder (C₁-C₆)-Alkyl steht,
sowie deren Salze, Hydrate, Hydrate der Salze und Solvate.

3. Verbindungen der Formel (I) nach Anspruch 1,
worin
R¹ für phenyl, Naphtyl, 5- bis 8-gliedriges Heteroaryl mit einem Stickstoff- oder Sauerstoffatom als Heteroatom und gegebenenfalls bis zu zwei weiteren Heteroatomen aus der Reihe N, O und/oder S oder 5-bis 8-gliedriges Heterocyclyl mit bis zu drei Heteroatomen aus der Reihe N, O und/oder S steht, wobei die Ringe ein- bis dreifach, unabhängig voneinander, durch Halogen, (C₁-C₆)-Alkyl, (C₁-C₆)-Alkoxy, Cyano, Trifluormethyl, Trifluormethoxy oder Trifluormethylthio substituiert sein können,
R² für einen Rest -N(R¹⁰)C(O)R¹¹ oder 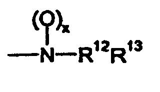 steht,
wobei
R¹⁰ und R¹¹, unabhängig voneinander, (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann, oder (C₃-C₇)-Cycloalkyl stehen,
oder
R¹⁰ und R¹¹ gemeinsam mit der N-C(O)-Gruppe, an die sie gebunden sind, einen 4- bis 7-gliedrigen Heterocyclus bilden, der bis zu zwei weitere Heteroatome aus der Reihe N, O und/oder S enthalten kann und der außerdem ein- bis dreifach, unabhängig voneinander, durch Halogen, (C₁-C₆)-Alkyl, Amino, Mono-oder Di-(C₁-C₆)-alkylamino, Hydroxy, (C₁-C₆)-Alkoxy, Trifluormethyl oder Cyano substituiert sein kann,
x für 0 oder 1 steht,
R¹² und R¹³ gemeinsam mit dem Stickstoffatom, an das sie gebunden sind, einen 4- bis 6-gliedrigen Heterocyclus bilden, der noch ein weiteres Heteroatom aus der Reihe N, O und/oder S enthalten kann und der einfach durch Amino, Hydroxy, Halogen, Trifluormethyl, Cyano, Oxo, Mono- oder Di-(C₁-C₄)- alkylamino, (C₁-C₄)-Alkoxy, Carboxamido, (C₁-C₄)-Alkylcarbonyl oder (C₃-C₅)-Cycloalkylcarbonyl substituiert sein kann,
R³ und R⁶, unabhängig voneinander, für Wasserstoff, Halogen, (C₁-C₆)-Alkyl, Amino, Mono- oder Di-(C₁-C₆)-alkylamino, Hydroxy, (C₁-C₆)- Alkoxy, (C₁-C₆)-Alkanoylamino, Cyano, Trifluormethyl, oder Nitro stehen,
R⁴ und R⁵ für Wasserstoff stehen,
und
R⁷ für Wasserstoff oder (C₁-C₄)-Alkyl steht,
sowie deren Salze, Hydrate, Hydrate der Salze und Solvate.

4. Verbindungen der Formel (I) nach Anspruch 1,
worin
R¹ für Phenyl, Furyl, Dihydrothienyl, Thiazolyl, Pyrrolyl oder Pyridyl steht, wobei die Ringe ein- bis dreifach, unabhängig voneinander, durch Fluor, Chlor, Brom, (C₁-C₄)-Alkyl, Trifluormethyl, Trifluormethoxy oder Trifluormethylthio substituiert sein können,
R² für einen Rest -N(R¹⁰)C(O)R¹¹ oder 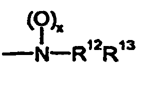 steht,
wobei
R¹⁰ und R¹¹, unabhängig voneinander, für (C₁-C₆)-Alkyl, das seinerseits durch Halogen, Amino, Mono- oder Di-(C₁-C₆)-alkylamino, Hydroxy, Oxo, (C₁-C₆)-Alkoxy, Trifluormethyl oder Cyano substituiert sein kann, oder (C₃-C₇)-Cycloalkyl stehen,
oder
R¹⁰ und R¹¹ gemeinsam mit der N-C(O)-Gruppe, an die sie gebunden sind, für Morpholinonyl, Pyrrolidinonyl, Thiomorpholinonyl oder Piperidinonyl stehen, wobei die Ringe ein- oder zweifach durch (C₁-C₄)-Alkyl substituiert sein können, x für 0 oder 1 steht,
R¹² und R¹³ gemeinsam mit dem Stickstoffatom, an das sie gebunden sind, einen 5- oder 6-gliedrigen gesättigten Heterocyclus bilden, der noch ein weiteres Sauerstoffatom im Ring enthalten kann und der einfach durch Amino oder Hydroxy substituiert sein kann,
R³ für Wasserstoff, Fluor, Chlor, Brom, (C₁-C₄)-Alkyl, Amino, Mono- oder Di-(C₁-C₃)-alkylamino, Cyano oder Nitro stehen,
R⁴, R⁵ und R⁶ für Wasserstoff stehen,
und
R⁷ für Wasserstoff steht,
sowie deren Salze, Hydrate, Hydrate der Salze und Solvate.

5. Verfahren zur Herstellung von Verbindungen der Formel (I) gemäß Anspruch 1, **dadurch gekennzeichnet, dass** man
Verbindungen der Formel (II) 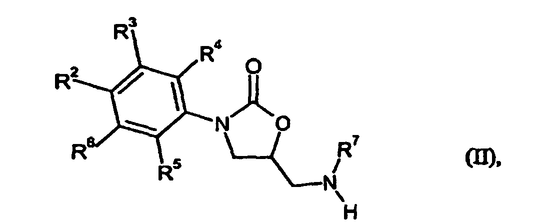 worin R², R³, R⁴, R⁵, R⁶ und R⁷ die oben angegebenen Bedeutungen haben,
mit Carbonsäuren der Formel (III) 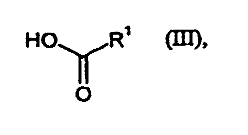 worin R¹ die oben angegebene Bedeutung hat,
oder aber mit den entsprechenden Carbonsäurehalogeniden oder aber mit den entsprechenden symmetrischen oder gemischten Carbonsäureanhydriden der zuvor definierten Carbonsäuren der Formel (III) umsetzt.

6. Verbindungen der Formel (I) gemäß Anspruch 1 zur Prävention und/oder Behandlung von Erkrankungen.

7. Arzneimittel enthaltend mindestens eine Verbindung der Formel (I) gemäß Anspruch 1 und mindestens einen weiteren Hilfsstoff.

8. Verwendung von Verbindungen der Formel (I) zur Herstellung eines Arzneimittels zur Prävention und/oder Behandlung von thromboembolischen Erkrankungen, insbesondere Herzinfarkt, Angina Pectoris (eingeschlossen instabile Angina), Reokklusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen.

9. Verwendung von Verbindungen der Formel (I) gemäß Anspruch 1 zur Herstellung eines Arzneimittels zur Prävention und/oder Behandlung der disseminierten intravasalen Gerinnung (DIC).

10. Verwendung von Verbindungen der Formel (I) gemäß Anspruch 1 zur Herstellung eines Arzneimittels zur Prävention und/oder Behandlung von Erkrankungen wie Atherosklerose; Arthritis; Alzheimer'sche Erkrankung oder Krebs.

11. Verfahren zur Verhinderung der Blutkoagulation in vitro, insbesondere bei Blutkonserven oder biologischen Proben, die Faktor Xa enthalten, **dadurch gekennzeichnet, dass** Verbindungen der allgemeinen Formel (I) gemäß Anspruch 1 zugegeben werden.

## Claims

1. Compounds of the formula (I) 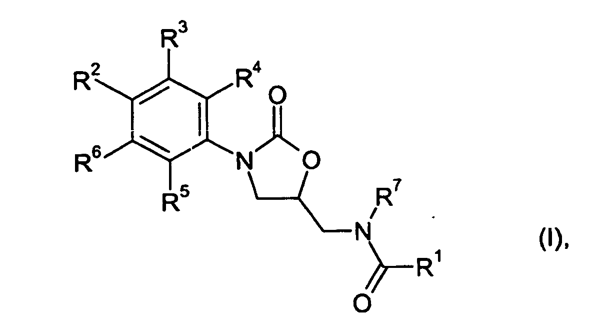 in which
R¹ represents (C₆-C₁₄)-aryl, 5- to 10-membered heteroaryl having up to three heteroatoms from the group consisting of N, O and S or 5- to 10-membered heterocyclyl having up to three heteroatoms from the group consisting of N, O and S, where the rings may be mono- to trisubstituted, independently of one another, by halogen, (C₁-C₆)-alkyl, amino, mono- or di-(C₁-C₆)-alkylamino, hydroxyl, (C₁-C₆)-alkoxy, (C₁-C₆)-alkoxycarbonyl, (C₁-C₆)-alkanoyl, trifluoromethyl, trifluoromethoxy, trifluoromethylthio, nitro, oxo, carboxyl or cyano,
R² represents a radical -N(R¹⁰)C(O)R¹¹ or 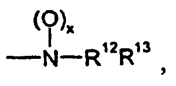
where
R¹⁰ and R¹¹, independently of one another, represent (C₁-C₆)-alkyl which for its part may be substituted by halogen, amino, mono- or di-(C₁-C₆)-alkylamino, hydroxyl, oxo, (C₁-C₆)-alkoxy, trifluoromethyl or cyano,
(C₆-C₁₄)-aryl which for its part may be substituted by halogen, (C₁-C₆)-alkyl, amino, mono- or di-(C₁-C₆)-alkylamino, hydroxyl, (C₁-C₆)-alkoxy, (C₁-C₆)-alkoxycarbonyl, (C₁-C₆)-alkanoyl, trifluoromethyl, trifluoromethoxy, trifluoromethylthio, nitro, carboxyl or cyano,
or (C₃-C₇)-cycloalkyl,
or
R¹⁰ and R¹¹ together with the N-C(O) group to which they are attached form a 4- to 7-membered heterocycle which may contain up to two further heteroatoms from the group consisting of N, O and S and which may furthermore be mono- to trisubstituted, independently of one another, by halogen, (C₁-C₆)-alkyl, amino, mono- or di-(C₁-C₆)-alkylamino, hydroxyl, (C₁-C₆)-alkoxy, trifluoromethyl or cyano,
x represents 0 or 1,
R¹² and R¹³ together with the nitrogen atom to which they are attached form a 4- to 6-membered heterocycle which may contain a further heteroatom from the group consisting of N, O and S and which may be up to disubstituted, independently of one another, by amino, hydroxyl, halogen, trifluoromethyl, cyano, oxo, mono- or di-(C₁-C₄)-alkylamino, (C₁-C₄)-alkoxy, carboxamido, (C₁-C₄)-alkylcarbonyl or (C₃-C₅)-cycloalkylcarbonyl,
R³, R⁴, R⁵ and R⁶, independently of one another, represent hydrogen, halogen, (C₁-C₆)-alkyl, amino, mono- or di-(C₁-C₆)-alkylamino, mono- or di-(C₁-C₆)-alkylaminocarbonyl, hydroxyl, (C₁-C₆)-alkoxy, (C₁-C₆)-alkanoyl, (C₁-C₆)-alkanoylamino, trifluoromethyl, carbamoyl, nitro or cyano,
and
R⁷ represents hydrogen or (C₁-C₆)-alkyl,
and its salts, hydrates, hydrates of the salts and solvates,
but excluding compounds of the general formula (I) in which the radical R¹ is an optionally substituted thiophene radical.

2. Compounds of the formula (I) according to Claim 1,
in which
R¹ represents (C₆-C₁₄)-aryl, 5- to 10-membered heteroaryl having one nitrogen or oxygen atom as heteroatom and optionally up to two further heteroatoms from the group consisting of N, O and S or 5- to 10-membered heterocyclyl having up to three heteroatoms from the group consisting of N, O and S, where the rings may be mono- to trisubstituted, independently of one another, by halogen, (C₁-C₆)-alkyl, amino, mono- or di-(C₁-C₆)-alkylamino, hydroxyl, (C₁-C₆)-alkoxy, (C₁-C₆)-alkoxycarbonyl, (C₁-C₆)-alkanoyl, trifluoromethyl, trifluoromethoxy, trifluoromethylthio, nitro, oxo, carboxyl or cyano,
R² represents a radical -N(R¹⁰)C(O)R¹¹ or 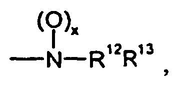
where
R¹⁰ and R¹¹, independently of one another, represent (C₁-C₆)-alkyl which for its part may be substituted by halogen, amino, mono- or di-(C₁-C₆)-alkylamino, hydroxyl, oxo, (C₁-C₆)-alkoxy, trifluoromethyl or cyano,
(C₆-C₁₄)-aryl which for its part may be substituted by halogen, (C₁-C₆)-alkyl, amino, mono- or di-(C₁-C₆)-alkylamino, hydroxyl, (C₁-C₆)-alkoxy, (C₁-C₆)-alkoxycarbonyl, (C₁-C₆)-alkanoyl, trifluoromethyl, trifluoromethoxy, trifluoromethylthio, nitro, carboxyl or cyano,
or (C₃-C₇)-cycloalkyl,
or
R¹⁰ and R¹¹ together with the N-C(O) group to which they are attached form a 4- to 7-membered heterocycle which may contain up to two further heteroatoms from the group consisting of N, O and S and which may furthermore be mono- to trisubstituted, independently of one another, by halogen, (C₁-C₆)-alkyl, amino, mono- or di-(C₁-C₆)-alkylamino, hydroxyl, (C₁-C₆)-alkoxy, trifluoromethyl or cyano,
x represents 0 or 1,
R¹² and R¹³ together with the nitrogen atom to which they are attached form a 4- to 6-membered heterocycle which may contain a further heteroatom from the group consisting of N, O and S and which may be up to disubstituted, independently of one another, by amino, hydroxyl, halogen, trifluoromethyl, cyano, oxo, mono- or di-(C₁-C₄)-alkylamino, (C₁-C₄)-alkoxy, carboxamido, (C₁-C₄)-alkylcarbonyl or (C₃-C₅)-cycloalkylcarbonyl,
R³, R⁴, R⁵ and R⁶, independently of one another, represent hydrogen, halogen, (C₁-C₆)-alkyl, amino, mono- or di-(C₁-C₆)-alkylamino, mono- or di-(C₁-C₆)-alkylaminocarbonyl, hydroxyl, (C₁-C₆)-alkoxy, (C₁-C₆)-alkanoyl, (C₁-C₆)-alkanoylamino, trifluoromethyl, carbamoyl, nitro or cyano,
and
R⁷ represents hydrogen or (C₁-C₆)-alkyl,
and their salts, hydrates, hydrates of the salts and solvates.

3. Compounds of the formula (I) according to Claim 1,
in which
R¹ represents phenyl, naphthyl, 5- to 8-membered heteroaryl having one nitrogen or oxygen atom as heteroatom and optionally up to two further heteroatoms from the group consisting of N, O and S or 5- to 8-membered heterocyclyl having up to three heteroatoms from the group consisting of N, O and S, where the rings may be mono- to trisubstituted, independently of one another, by halogen, (C₁-C₆)-alkyl, (C₁-C₆)-alkoxy, cyano, trifluoromethyl, trifluoromethoxy or trifluoromethylthio,
R² represents a radical -N(R¹⁰)C(O)R¹¹ or, 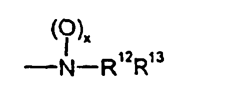
where
R¹⁰ and R¹¹, independently of one another, represent (C₁-C₆)-alkyl which for its part may be substituted by halogen, amino, mono- or di-(C₁-C₆)-alkylamino, hydroxyl, oxo, (C₁-C₆)-alkoxy, trifluoromethyl or cyano, or (C₃-C₇)-cycloalkyl,
or
R¹⁰ and R¹¹ together with the N-C(O) group to which they are attached form a 4- to 7-membered heterocycle which may contain up to two further heteroatoms from the group consisting of N, O and S and which may furthermore be mono- to trisubstituted, independently of one another, by halogen, (C₁-C₆)-alkyl, amino, mono- or di-(C₁-C₆)-alkylamino, hydroxyl, (C₁-C₆)-alkoxy, trifluoromethyl or cyano,
x represents 0 or 1,
R¹² and R¹³ together with the nitrogen atom to which they are attached form a 4- to 6-membered heterocycle which may contain a further heteroatom from the group consisting of N, O and S and which may be monosubstituted by amino, hydroxyl, halogen, trifluoromethyl, cyano, oxo, mono- or di-(C₁-C₄)-alkylamino, (C₁-C₄)-alkoxy, carboxamido, (C₁-C₄)-alkylcarbonyl or (C₃-C₅)-cycloalkylcarbonyl,
R³ and R⁶, independently of one another, represent hydrogen, halogen, (C₁-C₆)-alkyl, amino, mono- or di-(C₁-C₆)-alkylamino, hydroxyl, (C₁-C₆)-alkoxy, (C₁-C₆)-alkanoylamino, cyano, trifluoromethyl or nitro,
R⁴ and R⁵ represent hydrogen,
and
R⁷ represents hydrogen or (C₁-C₄)-alkyl,
and their salts, hydrates, hydrates of the salts and solvates.

4. Compounds of the formula (I) according to Claim 1,
in which
R¹ represents phenyl, furyl, dihydrothienyl, thiazolyl, pyrrolyl or pyridyl, where the rings may be mono- to trisubstituted, independently of one another, by fluorine, chlorine, bromine, (C₁-C₄)-alkyl, trifluoromethyl, trifluoromethoxy or trifluoromethylthio,
R² represents a radical -N(R¹⁰)C(O)R¹¹ or 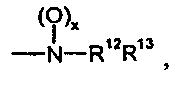
where
R¹⁰ and R¹¹, independently of one another, represent (C₁-C₆)-alkyl which for its part may be substituted by halogen, amino, mono- or di-(C₁-C₆)-alkylamino, hydroxyl, oxo, (C₁-C₆)-alkoxy, trifluoromethyl or cyano, or (C₃-C₇)-cycloalkyl,
or
R¹⁰ and R¹¹ together with the N-C(O) group to which they are attached represent morpholinonyl, pyrrolidinonyl, thiomorpholinonyl or piperidinonyl, where the rings may be mono- or disubstituted by (C₁-C₄)-alkyl,
x represents 0 or 1,
R¹² and R¹³ together with the nitrogen atom to which they are attached form a 5- or 6-membered saturated heterocycle which may contain a further oxygen atom in the ring and which may be monosubstituted by amino or hydroxyl,
R³ represents hydrogen, fluorine, chlorine, bromine, (C₁-C₄)-alkyl, amino, mono- or di-(C₁-C₃)-alkylamino, cyano or nitro,
R⁴, R⁵ and R⁶ represent hydrogen,
and
R⁷ represents hydrogen,
and their salts, hydrates, hydrates of the salts and solvates.

5. Process for preparing compounds of the formula (I) according to Claim 1,
**characterized in that**
compounds of the formula (II) 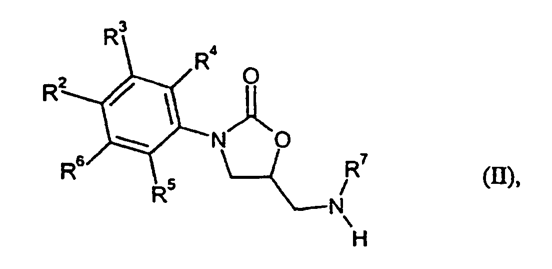 in which R², R³, R⁴, R⁵, R⁶ and R⁷ are as defined above
are reacted with carboxylic acids of the formula (III) 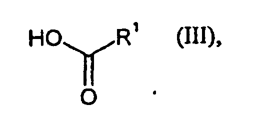 in which R¹ is as defined above,
or else with the corresponding carbonyl halides or else with the corresponding symmetric or mixed carboxylic anhydrides of the carboxylic acids of the formula (III) defined above.

6. Compounds of the formula (I) according to Claim 1 for the prevention and/or treatment of disorders.

7. Medicaments comprising at least one compound of the formula (I) according to Claim 1 and at least one further auxiliary.

8. The use of compounds of the formula (I) for preparing a medicament for the prevention and/or treatment of thromboembolic disorders, in particular myocardial infarction, angina pectoris (including unstable angina), reocclusions and restenoses after angioplasty or aortocoronary bypass, cerebro vascular accident, transitory ischemic attacks, peripheral occlusive diseases, pulmonary embolisms or deep vein thromboses.

9. The use of compounds of the formula (I) according to Claim 1 for preparing a medicament for the prevention and/or treatment of disseminated intravasal coagulation (DIC).

10. The use of compounds of the formula (I) according to Claim 1 for preparing a medicament for the prevention and/or treatment of disorders such as atherosclerosis; arthritis; Alzheimer's disease or cancer.

11. Process for preventing the coagulation of blood in vitro, in particular in banked blood or biological samples containing factor Xa, **characterized in that** compounds of the general formula (I) according to Claim 1 are added.

## Revendications

1. Composés de formule (I) 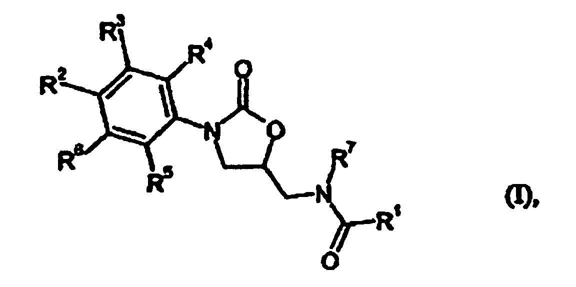 dans laquelle
R¹ est un reste aryle en C₆ à C₁₄, un reste hétéroaryle pentagonal à décagonal ayant jusqu'à trois hétéroatomes de la série N, O et/ou S ou un reste hétérocyclyle pentagonal à décagonal ayant jusqu'à trois hétéroatomes de la série N, O et/ou S, les noyaux pouvant porter un à trois substituants, indépendamment les uns des autres, halogéno, alkyle en C₁ à C₆, amino, mono- ou di(alkyle en C₁ à C₆)amino, hydroxy, alkoxy en C₁ à C₆, (alkoxy en C₁ à C₆)carbonyle, alcanoyle en C₁ à C₆, trifluorométhyle, trifluorométhoxy, trifluorométhylthio, nitro, oxo, carboxyle ou cyano,
R² représente un reste -N(R¹⁰)C(O)R¹¹ ou 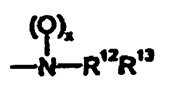
dans lequel
R¹⁰ et R¹¹ représentent indépendamment l'un de l'autre un reste alkyle en C₁ à C₆ qui peut être lui-même substitué par un radical halogéno, amino, mono- ou di(alkyle en C₁ à C₆)amino, hydroxy, oxo, alkoxy en C₁ à C₆, trifluorométhyle ou cyano,
un reste aryle en C₆ à C₁₄ qui peut lui-même être substitué par un radical halogéno, alkyle en C₁ à C₆, amino, mono- ou di(alkyle en C₁ à C₆)amino, hydroxy, alkoxy en C₁ à C₆, (alkoxy en C₁ à C₆)carbonyle, alcanoyle en C₁ à C₆, trifluorométhyle, trifluorométhoxy, trifluorométhylthio, nitro, carboxyle ou cyano,
ou un reste cycloalkyle en C₃ à C₇,
ou bien
R¹⁰ et R¹¹ forment conjointement avec le groupe N-C(O) auquel ils sont liés un hétérocycle tétragonal à heptagonal qui peut contenir jusqu'à deux autres hétéroatomes de la série N, O et/ou S et qui peut en outre porter un à trois substituants, indépendamment les uns des autres, halogéno, alkyle en C₁ à C₆, amino, mono- ou di(alkyle en C₁ à C₆)amino, hydroxy, alkoxy en C₁ à C₆, trifluorométhyle ou cyano,
x a la valeur 0 ou 1,
R¹² et R¹³ forment, conjointement avec l'atome d'azote auquel ils sont liés, un hétérocycle tétragonal à hexagonal qui peut contenir encore un autre hétéroatome de la série N, O et/ou S et qui peut porter un ou deux substituants, indépendants l'un de l'autre, amino, hydroxy, halogéno, trifluorométhyle, cyano, oxo, mono- ou di(alkyle en C₁ à C₄)amino, alkoxy en C₁ à C₄, carboxamido, (alkyle en C₁ à C₄)carbonyle ou (cycloalkyle en C₃ à C₅)carbonyle,
R³, R⁴, R⁵ et R⁶ représentent, indépendamment les uns de autres, l'hydrogène, un halogène, un reste alkyle en C₁ à C₆, un groupe amino, un reste mono- ou di(alkyle en C₁ à C₆)amino, mono- ou di (alkyle en C₁ à C₆)aminocarbonyle, un groupe hydroxy, un reste alkoxy en C₁ à C₆, alcanoyle en C₁ à C₆, alcanoylamino en C₁ à C₆, trifluorométhyle, carbamoyle, un groupe nitro ou cyano,
et
R⁷ représente l'hydrogène ou un reste alkyle en C₁ à C₆, ainsi que leurs sels, leurs hydrates, les hydrates de leurs sels et leurs produits de solvatation, excepté toutefois des composés de formule générale (I) dans lesquels le reste R¹ est un reste thiophène éventuellement substitué.

2. Composés de formule (I) suivant la revendication 1,
formule dans laquelle
R¹ est un reste aryle en C₆ à C₁₄, un reste hétéroaryle pentagonal à décagonal ayant comme hétéroatome un atome d'azote ou d'oxygène et, le cas échéant, jusqu'à deux autres hétéroatomes de la série N, O et/ou S, ou un reste hétérocyclyle pentagonal à décagonal ayant jusqu'à trois hétéroatomes de la série N, O et/ou S, les noyaux pouvant porter un à trois substituants, indépendamment les uns des autres, halogéno, alkyle en C₁ à C₆, amino, mono- ou di(alkyle en C₁ à C₆)amino, hydroxy, alkoxy en C₁ à C₆, (alkoxy en C₁ à C₆)carbonyle, alcanoyle en C₁ à C₆, trifluorométhyle, trifluorométhoxy, trifluorométhylthio, nitro, oxo, carboxyle ou cyano,
R² représente un reste -N(R¹⁰)C(O)R¹¹ ou 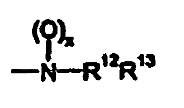
dans lequel
R¹⁰ et R¹¹ représentent, indépendamment l'un de l'autre, un reste alkyle en C₁ à C₆ qui peut lui-même être substitué par un radical halogéno, amino, mono- ou di(alkyle en C₁ à C₆)amino, hydroxy, oxo, alkoxy en C₁ à C₆, trifluorométhyle ou cyano,
un reste aryle en C₆ à C₁₄, qui peut lui-même être substitué par un radical halogéno, alkyle en C₁ à C₆, amino, mono- ou di(alkyle en C₁ à C₆)amino, hydroxy, alkoxy en C₁ à C₆, (alkoxy en C₁ à C₆)carbonyle, alcanoyle en C₁ à C₆, trifluorométhyle, trifluorométhoxy, trifluorométhylthio, nitro, carboxyle ou cyano,
ou un reste cycloalkyle en C₃ à C₇,
ou bien
R¹⁰ et R¹¹ forment, conjointement avec le groupe N-C(O) auquel ils sont liés, un hétérocycle tétragonal à heptagonal qui peut contenir jusqu'à deux autres hétéroatomes de la série N, O et/ou S et qui peut en outre porter un à trois substituants, indépendamment les uns des autres, halogéno, alkyle en C₁ à C₆, amino, mono- ou di(alkyle en C₁ à C₆)amino, hydroxy, alkoxy en C₁ à C₆, trifluorométhyle ou cyano,
x a la valeur 0 ou 1,
R¹² et R¹³ forment, conjointement avec l'atome d'azote auquel ils sont liés, un hétérocycle tétragonal à hexagonal qui peut contenir encore un autre hétéroatome de la série N, O et/ou S et qui peut porter un ou deux substituants, indépendants l'un de l'autre, amino, hydroxy, halogéno, trifluorométhyle, cyano, oxo, mono- ou di(alkyle en C₁ à C₄)amino, alkoxy en C₁ à C₄, carboxamido, (alkyle en C₁ à C₄)carbonyle ou (cycloalkyle en C₃ à C₅) carbonyle,
R³, R⁴, R⁵ et R⁶ représentent, indépendamment les uns des autres, l'hydrogène, un halogène, un reste alkyle en C₁ à C₆, un groupe amino, un reste mono- ou di(alkyle en C₁ à C₆)amino, mono- ou di (alkyle en C₁ à C₆)aminocarbonyle, un groupe hydroxy, un reste alkoxy en C₁ à C₆, alcanoyle en C₁ à C₆, alcanoylamino en C₁ à C₆, trifluorométhyle, carbamoyle, un groupe nitro ou cyano,
et
R⁷ représente l'hydrogène ou un reste alkyle en C₁ à C₆, ainsi que leurs sels, leurs hydrates, les hydrates de leurs sels et leurs produits de solvatation.

3. Composés de formule (I) suivant la revendication 1,
formule dans laquelle
R¹ est un reste phényle, naphtyle, un reste hétéroaryle pentagonal à octogonal ayant comme hétéroatome un atome d'azote ou d'oxygène et, le cas échéant, jusqu'à deux autres hétéroatomes de la série N, O et/ou S ou un reste hétérocyclyle pentagonal à octogonal ayant jusqu'à trois hétéroatomes de la série N, 0 et/ou S, les noyaux pouvant porter un à trois substituants, indépendamment les uns des autres, halogéno, alkyle en C₁ à C₆, alkoxy en C₁ à C₆, cyano, trifluorométhyle, trifluorométhoxy ou trifluorométhylthio,
R² est un reste -N(R¹⁰)C(O)R¹¹ ou 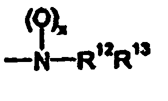
dans lequel
R¹⁰ et R¹¹ représentent, indépendamment l'un de l'autre, un reste alkyle en C₁ à C₆ qui peut lui-même porter un substituant halogéno, amino, mono-ou di(alkyle en C₁ à C₆)amino, hydroxy, oxo, alkoxy en C₁ à C₆, trifluorométhyle ou cyano, ou un reste cycloalkyle en C₃ à C₇,
ou bien
R¹⁰ et R¹¹ forment, conjointement avec le groupe N-C(O) auquel ils sont liés, un hétérocycle tétragonal à heptagonal qui peut contenir jusqu'à deux autres hétéroatomes de la série N, O et/ou S et qui peut en outre porter un à trois substituants, indépendamment les uns des autres, halogéno, alkyle en C₁ à C₆, amino, mono- ou di(alkyle en C₁ à C₆) amino, hydroxy, alkoxy en C₁ à C₆, trifluorométhyle ou cyano,
x a la valeur 0 ou 1,
R¹² et R¹³ forment, conjointement avec l'atome d'azote auquel ils sont liés, un hétérocycle tétragonal à hexagonal qui peut contenir encore un autre hétéroatome de la série N, O et/ou S et qui peut être substitué une fois par un radical amino, hydroxy, halogéno, trifluorométhyle, cyano, oxo, mono- ou di(alkyle en C₁ à C₄)amino, alkoxy en C₁ à C₄, carboxamido, (alkyle en C₁ à C₄)carbonyle ou (cycloalkyle en C₃ à C₅)carbonyle,
R³ et R⁶ représentent, indépendamment l'un de l'autre, l'hydrogène, un halogène, un reste alkyle en C₁ à C₆, un groupe amino, un reste mono- ou di(alkyle en C₁ à C₆)amino, un groupe hydroxy, un reste alkoxy en C₁ à C₆, alcanoylamino en C₁ à C₆, un groupe cyano, un reste trifluorométhyle ou un groupe nitro,
R⁴ et R⁵ représentent l'hydrogène,
et
R⁷ représente l'hydrogène ou un reste alkyle en C₁ à C₄, ainsi que leurs sels, leurs hydrates, les hydrates de leurs sels et leurs produits de solvatation.

4. Composés de formule (I) suivant la revendication 1,
formule dans laquelle
R¹ est un reste phényle, furyle, dihydrothiényle, thiazolyle, pyrrolyle ou pyridyle, les noyaux pouvant porter un à trois substituants, indépendamment les uns des autres, fluoro, chloro, bromo, alkyle en C₁ à C₄, trifluorométhyle, trifluorométhoxy ou trifluorométhylthio,
R² représente un reste -N(R¹⁰)C(O)R¹¹ ou 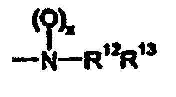
dans lequel
R¹⁰ et R¹¹ représentent, indépendamment l'un de l'autre, un reste alkyle en C₁ à C₆ qui peut lui-même être substitué par un radical halogéno, amino, mono- ou di(alkyle en C₁ à C₆)amino, hydroxy, oxo, alkoxy en C₁ à C₆, trifluorométhyle ou cyano, ou un reste cycloalkyle en C₃ à C₇,
ou bien
R¹⁰ et R¹¹ forment, conjointement avec le groupe N-C(O) auquel ils sont liés, un reste morpholinonyle, pyrrolidinonyle, thiomorpholinonyle ou pipéridinonyle, les noyaux pouvant être substitués une ou deux fois par un radical alkyle en C₁ à C₄,
x a la valeur 0 ou 1,
R¹² et R¹³ forment, conjointement avec l'atome d'azote auquel ils sont liés, un hétérocycle saturé pentagonal ou hexagonal qui peut contenir encore un autre atome d'oxygène dans le noyau et qui peut être substitué une fois par un radical amino ou hydroxy,
R³ représente l'hydrogène, le fluor, le chlore, le brome, un reste alkyle en C₁ à C₄, un groupe amino, un reste mono- ou di(alkyle en C₁ à C₃)amino, un groupe cyano ou nitro,
R⁴, R⁵ et R⁶ représentent l'hydrogène,
et
R⁷ représente l'hydrogène,
ainsi que leurs sels, leurs hydrates, les hydrates de leurs sels et leurs produits de solvatation.

5. Procédé de production de composés de formule (I) suivant la revendication 1, **caractérisé en ce qu'**on fait réagir
des composés de formule (II) 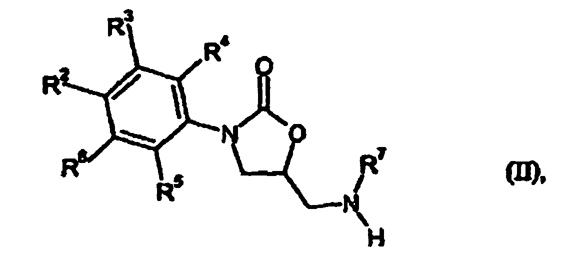 dans laquelle R², R³, R⁴, R⁵, R⁶ et R⁷ ont les définitions indiquées ci-dessus,
avec des acides carboxyliques de formule (III) 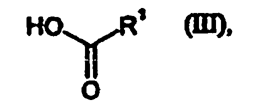 dans laquelle R¹ a la définition indiquée ci-dessus,
ou bien avec les halogénures d'acides carboxyliques correspondants ou avec les anhydrides d'acides carboxyliques symétriques ou mixtes correspondants des acides carboxyliques de formule (III) définis ci-dessus.

6. Composés de formule (I) suivant la revendication 1, destinés à la prévention et/ou au traitement de maladies.

7. Médicament contenant au moins un composé de formule (I) suivant la revendication 1, et au moins une autre substance auxiliaire.

8. Utilisation de composés de formule (I) pour la préparation d'un médicament destiné à la prévention et/ou au traitement de maladies thromboemboliques, en particulier de l'infarctus du myocarde, de l'angine de poitrine (y compris l'angor instable), de réocclusions et de resténoses après une angioplastie ou un pontage aorto-coronarien, l'apoplexie cérébrale, des attaques ischémiques transitoires, des artériopathies périphériques oblitérantes, des embolies pulmonaires ou des thromboses veineuses profondes.

9. Utilisation de composés de formule (I) suivant la revendication 1, pour la préparation d'un médicament destiné à prévenir et/ou à traiter la coagulation intravasculaire disséminée (DIC).

10. Utilisation de composés de formule (I) suivant la revendication 1, pour la préparation d'un médicament destiné à prévenir et/ou à traiter des maladies telles que l'athérosclérose, l'arthrite, la maladie d'Alzheimer ou le cancer.

11. Procédé pour empêcher la coagulation sanguine in vitro, notamment dans des conserves de sang ou des échantillons biologiques qui contiennent le facteur Xa, **caractérisé en ce qu'**on ajoute des composés de formule générale (I) suivant la revendication 1.
